# EUROPÄISCHE PATENTANMELDUNG

(11) **EP 3 415 007 A1**
(43) Veröffentlichungstag der Anmeldung: **19.12.2018**
(21) Anmeldenummer: 17175390.8
(22) Anmeldetag: 12.06.2017
(51) Int. Cl.: A01N 43/653, A01N 31/14, A01N 37/06, A01N 41/10

(54) **PTZ FORMULIERUNGEN MIT NIEDRIGEM GEHALT AN DESTHIO**

(71) Anmelder: Bayer AG, 51373 Leverkusen (DE)
(72) Erfinder: Die Erfindernennung liegt noch nicht vor
(74) Vertreter: BIP Patents

(57) **Zusammenfassung**

Die Erfindung betrifft lagerstabile Prothioconazol-haltige Formulierungen mit einem besonders niedrigem Gehalt an 2-(1-Chlorocyclopropyl)-1-(2-chlorophenyl)-3-(1H-1,2,4-triazol-1-yl)propan-2-ol auf Basis von organischen Vinylverbindungen, ein Verfahren zu deren Herstellung, ein Verfahren zur Bekämpfung phytopathogener Pilze im Pflanzenschutz, sowie deren Verwendung als Pflanzenschutzmittel.

## Beschreibung

Die Erfindung betrifft lagerstabile Prothioconazol-haltige Formulierungen mit einem besonders niedrigem Gehalt an 2-(1-Chlorocyclopropyl)-1-(2-chlorophenyl)-3-(1H-1,2,4-triazol-1-yl)propan-2-ol auf Basis von organischen Vinylverbindungen, ein Verfahren zu deren Herstellung, ein Verfahren zur Bekämpfung phytopathogener Pilze im Pflanzenschutz, sowie deren Verwendung als Pflanzenschutzmittel.

Es ist bereits bekannt, dass Prothioconazol in üblichen Formulierungen zur Bekämpfung von Pilzen verwendet werden kann (WO-A 96/16 048). Bei diesem Wirkstoff handelt es sich um das 2-[2-(1-Chlor-cyclopropyl)-3-(2-chlor-phenyl)-2-hydroxy-propyl]-2,4-dihydro-3H-1,2,4-triazol-3-thion. Prothioconazole-haltige Formulierungen sind im allgemeinen flüssige Formulierungen und werden beispielsweise als Emulsionskonzentrate auf dem Markt angeboten.

Es ist bekannt, dass sich der Wirkstoff Prothioconazole unter bestimmten Bedingungen zu der Verbindung 2-(1-Chlorocyclopropyl)-1-(2-chlorophenyl)-3-(1H-1,2,4-triazol-1-yl)propan-2-ol abbauen kann (PSM-Zulassungsbereicht, Tilmor, 2010.08.30, Lfd. Nr. 21, Bundesamt für Verbraucherschutz und Lebensmittelsicherheit).

Daher können Prothioconazol (im Folgenden PTZ genannt)-haltige Formulierungen bereits bei der Herstellung eine gewisse Menge an 2-(1-Chlorocyclopropyl)-1-(2-chlorophenyl)-3-(1H-1,2,4-triazol-1-yl)propan-2-ol enthalten. Bei der Lagerung unter drastischen Bedingungen, wie erhöhten Temperaturen, Licht-Einstrahlung sowie intensiver Sauerstoffkontakt, kann ebenfalls ein Abbau von Prothioconazole zu 2-(1-Chlorocyclopropyl)-1-(2-chlorophenyl)-3-(1H-1,2,4-triazol-1-yl)propan-2-ol stattfinden, wodurch sich der Anteil an Wirkstoff in den Formulierungen entsprechend verringert. Da es sich bei der Verbindung 2-(1-Chlorocyclopropyl)-1-(2-chlorophenyl)-3-(1H-1,2,4-triazol-1-yl)propan-2-ol (im Folgenden Desthio genannt) um eine relevante Verunreinigung handelt, ist deren Gehalt in Prothioconazol-haltigen Formulierungen regulatorisch limitiert.

Hierbei richtet sich die maximal zulässige Menge an Desthio in PTZ Formulierungen nach der Menge an PTZ in der Formulierung in g/L. Die maximal zulässige Menge an Desthio ergibt sich in ppm, wobei sich der Zulässige Anteil an Desthio in ppm als die Hälfte des Wertes der PTZ-Beladung in g/L ergibt. Der maximal zulässige Gehalt an Desthio in einer Formulierung mit 100 g/L PTZ ist somit 50 ppm (=0.005 wt.-%).

In der WO-A 2012/033590 werden wässrige Dispersionen von Prothioconazole offenbart, die eine schwefelhaltige Verbindung, wie beispielsweise L-Cysteine, zur Stabilisierung enthalten.

Die unveröffentlichte PCT/EP 2016/080215 (Bayer CropScience AG) wird als nächstliegender Stand der Technik angesehen. Hier werden Emulsionskonzentrate mit in einem Lösemittel gelöstem PTZ beschrieben. Die Formulierung konnte durch durch Zugabe von Formel (I) 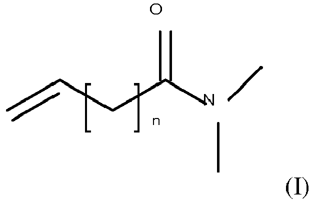 in welcher
- n: für 3, 4, 5, 6, 7, 8, 9, 10, 11, 12, 15 oder 18 steht
stabilisiert und eine Reduzierung des Gehalts an Desthio erreicht werden.

Verbindungen der Formel (I) sind aus der WO-A 2012/061094 bekannt und können über das Verfahren der Metathese hergestellt werden. Es handelt sich beispielsweise um Produkte der Firma Stepan ((N,N-Dimethyl 9-decenamid, CAS Nummer: 1356964-77-6, Hallcomid^{®} 1025 bzw. Steposol^{®} MET-10U).

Nachteilig an diesen Verbindungen ist, dass sie nur über einen technisch aufwändigen Olefinmetathese Schritt hergestellt werden, unerwünschte Metallverunreinigungen enthalten, die die Zersetzung von aktiven und nichtaktiven Inhaltsstoffen katalysieren können, und Verbindung I ausschließlich als Lösemittel verwendet werden kann. Somit sind alternative Formuliertypen, in denen PTZ nicht gelöst ist (wie etwas WG, SC, OD) nicht zugänglich. Nachteilig ist auch die limitierte Verfügbarkeit an Produkten. Aktuell gibt es in technischer Menge von Verbindung (I) nur Hallcomid 1025.

WO 2016/092030 beschreibt Zuckertenside mit 9-Decenoylrest zur Verwendung in wäßrigen Adjuvantzusammensetzungen zur Erhöhung der Wirksamkeit von Elektrolytwirkstoffen. Im folgenden werden die in der WO 2016/092030 beschriebenen Verbindungen als Glycosid-Tenside mit 9-Decenoylrest bezeichnet.

Es bestand somit Bedarf an stabilen PTZ-haltigen Formulierungen, die auch über längere Zeiträume und unter ungünstigen Lagerbedingungen, wie beispielweise Sauerstoffkontakt, hohen Temperaturen oder Lichteinfall, keine Mengen am Abbauprodukten des PTZ, insbesondere an 2-(1-Chlorocyclopropyl)-1-(2-chlorophenyl)-3-(1H-1,2,4-triazol-1-yl)propan-2-ol, aufweisen.

Aufgabe der vorliegenden Erfindung bestand somit in der Bereitstellung von neuen, verbesserten Prothioconazole-haltigen Formulierungen, die über eine hohe Lagerstabilität verfügen und keine wesentliche Abbauraten von Prothioconazole zu 2-(1-Chlorocyclopropyl)-1-(2-chlorophenyl)-3-(1H-1,2,4-triazol-1-yl)propan-2-ol zeigen, wobei die Formulierungen frei von Verbindungen gemäß Formel (I) sind.

Darüber hinaus sind die Formulierungen bevorzugt frei von Glycosid-Tensiden mit 9-Decenoylrest.

Gegenstand der vorliegenden Erfindung sind daher Formulierungen enthaltend
a) Prothioconazole sowie
b) mindestens eine Verbindung der Formel (IV) 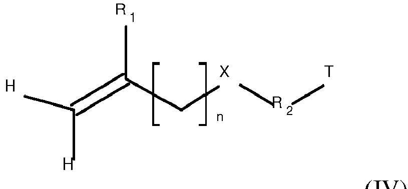 wobei
   n = 0 - 24; bevorzugt 2-16; weiter bevorzugt 4-14; und besonders bevorzugt 8-12:
   R₁ = H, C₁-C₆ Alkyl, -CN, Cl, Br, Fl, ; bevorzugt H, C₁-C₄ Alkyl, weiter bevorzugt H, Methyl; und besonders bevorzugt H;
   X= -COO-, -CONR₃R₄, -S-, -SO₂-, -O- und -COS-; bevorzugt -COO-, CONR₃R₄, -S-, -O- und - COS-; weiter bevorzugt -COO-, CONR₃R₄; und besonders bevorzugt -COO-,;
   R₂ = (-CH₂-)ₘ, (-CH₂O-)ₘ, (-C₂H₄O-)ₘ, (-C₃H₆O-)ₘ, (-C₄H₈O-)ₘ; bevorzugt (-CH₂-)ₘ, (-CH₂O-)ₘ, (-C₂H₄O-)ₘ, (-C₃H₆O-)ₘ; weiter bevorzugt (-CH₂O-)ₘ, (-C₂H₄O-)ₘ, (-C₃H₆O-)ₘ; und besonders bevorzugt (-CH₂O-)ₘ,
   T= H, Sulfat, Phosphat, C₁-C₃ Alkyl, -OH, -SH, Sulfon; bevorzugt H, Sulfat, Sulfon, -OH, Phosphat; weiter bevorzugt H, Sulfat, -OH; und besonders bevorzugt H und OH;
      R₃ = H, C₁-C₆ Alkyl, C₃-C₆ Cycloalkyl, C₆-C₁₀ Aryl, bevorzugt H, C₁-C₄ Alkyl, C5-C6 Cycloalkyl, Phenyl, weiter bevorzugt H, Methyl und Phenyl; und besonders bevorzugt H;
      R₄ = H, methyl, ethyl propyl, (-CH₂-)ₘ, (-CH₂O-)ₘ, (-C₂H₄O-)ₘ, (-C₃H₆O-)ₘ, (-C₄H₈O-)ₘ; bevorzugt H, methyl, ethyl propyl, (-CH₂-)ₘ, (-CH₂O-)ₘ, (-C₂H₄O-)ₘ, (-C₃H₆O-)ₘ; weiter bevorzugt
   H, methyl, ethyl propyl, (-CH₂O-)ₘ, (-C₂H₄O-)ₘ, (-C₃H₆O-)ₘ; und besonders bevorzugt H, methyl, ethyl propyl, (-CH₂O-)ₘ,
      und R₃ verschieden von R₄ ist, und
      m = 0 - 100, bevorzugt 2 - 50, weiter bevorzugt 5 - 25, und besonders bevorzugt 8 - 12;
   wobei die Formulierung frei von Verbindungen gemäß Formel (I) ist 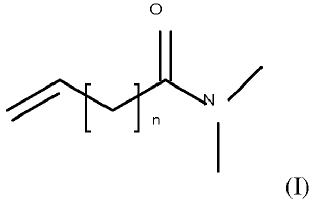 in welcher
   - n: für 3, 4, 5, 6, 7, 8, 9, 10, 11, 12, 15 oder 18 steht,
   und darüber hinaus frei von Glycosid-Tensiden mit 9-Decenoylrest,
   wobei
   bei X= -CNR₃R₄ und m=0 T entfällt.

In den oben genannten Definitionen der Reste, wie z.B. in C₃H₆O, auch die unter diese Summenformel fallende Strukturisomere umfaßt, die der Fachmann ohne weiteres erschließen kann. Ferner sind unter Begriffen wie " Sulfat, Phosphat,etc" die jeweiligen Reste als Radikal zu verstehen, wobei die entsprechenden Säuren auch als Salze vorliegen können. Als Gegen-Ion kommen hierzu vorzugsweise Alkalimetallionen, Calcium, Isopropylammonium und Ammonium in Betracht, es können aber auch andere dem Fachmann Gegenionen verwendet werden.

In einer bevorzugten Ausführungsform ist Gegenstand der vorliegenden Erfindung eine Formulierung enthaltend
a) Prothioconazole sowie
b) mindestens eine Verbindung der Formel (IV) 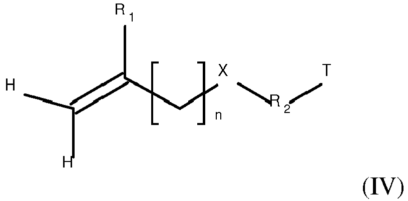 wobei
   n = 2-16;
   R₁ = H, C₁-C₄ Alkyl,
   X= -COO-, CONR₃R₄, -S-, -O- und -COS-; R₂ = (-CH₂-)ₘ, (-CH₂O-)ₘ, (-C₂H₄O-)ₘ, (-C₃H₆O-)ₘ;
   T= H, Sulfat, Sulfon, -OH, Phosphat;
      R₃ = H, C₁-C₄ Alkyl, C5-C6 Cycloalkyl, Phenyl;
      R₄ = H, methyl, ethyl propyl, (-CH₂-)ₘ, (-CH₂O-)ₘ, (-C₂H₄O-)ₘ, (-C₃H₆O-)ₘ;
      und R₃ verschieden von R₄ ist, und
      m=0 - 50;
   wobei die Formulierung frei von Verbindungen gemäß Formel (I) ist 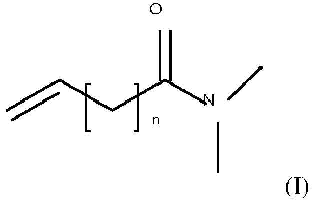 in welcher
   - n: für 3, 4, 5, 6, 7, 8, 9, 10, 11, 12, 15 oder 18 steht,
   und darüber hinaus frei von Glycosid-Tensiden mit 9-Decenoylrest,
   wobei
   bei X= -CNR₃R₄ und m=0 T entfällt.

In einer weiter bevorzugten Ausführungsform ist Gegenstand der vorliegenden Erfindung eine Formulierungen enthaltend
a) Prothioconazole sowie
b) mindestens eine Verbindung der Formel (IV) 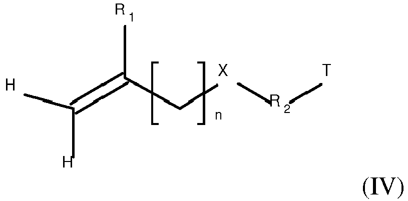 wobei
   n = 4-14;
   R₁ = H, Methyl; X= -COO-, CONR₃R₄;
   R₂ = (-CH_{2O}-)ₘ, (-C₂H₄O-)ₘ, (-C₃H₆O-)ₘ;
   T= H, Sulfat, -OH;
      R₃ = H, Methyl und Phenyl;
      R₄ = H, methyl, ethyl propyl, (-CH₂O-)ₘ, (-C₂H₄O-)ₘ, (-C₃H₆O-)ₘ;
      und R₃ verschieden von R₄ ist, und
      m=0-25,
   wobei die Formulierung frei von Verbindungen gemäß Formel (I) ist 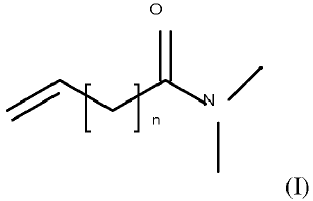 in welcher
   - n: für 3, 4, 5, 6, 7, 8, 9, 10, 11, 12, 15 oder 18 steht,
   und darüber hinaus frei von Glycosid-Tensiden mit 9-Decenoylrest,
   wobei
   bei X= -CNR₃R₄ und m=0 T entfällt.

In einer besonders bevorzugten Ausführungsform ist Gegenstand der vorliegenden Erfindung eine Formulierungen enthaltend
a) Prothioconazole sowie
b) mindestens eine Verbindung der Formel (IV) 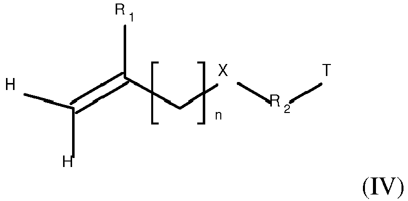 wobei
   n = 8-12:
   R₁ = H;
   X = -COO-,
   R₂ = (-CH₂O-)ₘ,
   T= H und OH;
      m=8-12;
   wobei die Formulierung frei von Verbindungen gemäß Formel (I) ist 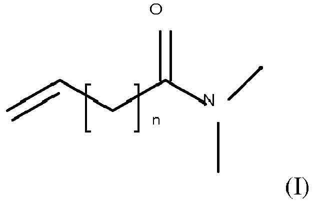 in welcher
   - n: für 3, 4, 5, 6, 7, 8, 9, 10, 11, 12, 15 oder 18 steht,
   und darüber hinaus frei von Glycosid-Tensiden mit 9-Decenoylrest.

In einer alternativen bevorzugten Ausführungsform mit n = 7 und m = 0 ist Gegenstand der vorliegenden Erfindung eine Formulierung enthaltend
a) Prothioconazole sowie
b) mindestens eine Verbindung der Formel (IV) 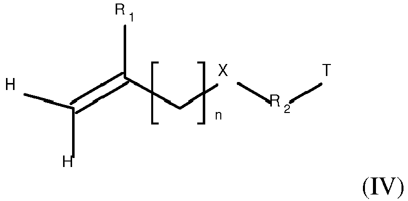 wobei
   n =7:
   R₁ = H;
   X = -COO-,;
   T=H;
      m=0;
   wobei die Formulierung frei von Verbindungen gemäß Formel (I) ist 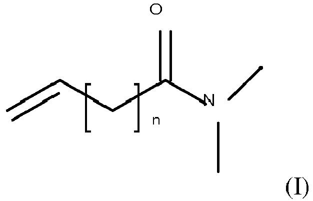 in welcher
   - n: für 3, 4, 5, 6, 7, 8, 9, 10, 11, 12, 15 oder 18 steht,
   und darüber hinaus frei von Glycosid-Tensiden mit 9-Decenoylrest sind.

In den zuvor genannten Ausführungsformen kann R₂ auch ein Copolymer, vorzugsweise ein Blockcopolymer aus mindestens zwei Monomeren ausgewählt aus (-CH₂-)ₘ, (-CH₂O-)ₘ, (-C₂H₄O-)ₘ, (-C₃H₆O-)ₘ, (-C₄H₈O-)ₘ sein, vorzugsweise ist R₂ dann ein Blockcopolymer aus (-C₂H₄O-)ₘ, (-C₃H₆O-)ₘ.

In einer bevorzugten Ausführungsform ist die Verbindung IV ausgewählt aus der Gruppe, die 9-Decensäure, 9-Decenoatester mit n EO Gruppen (EO= C₂H₄O), wobei n= 8,9,10,11, und bevorzugt n=10 ist;
C10-17 DMAPA-Amide 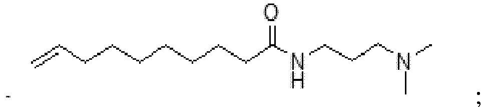 C10-20 DMAPA Amine-Oxide 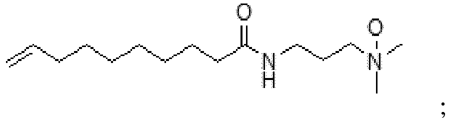 und
C10-24: Sulfobetaine 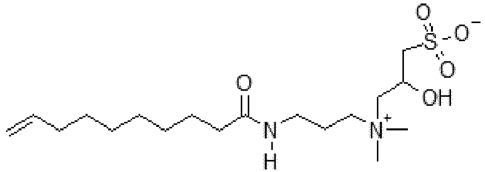 umfaßt.

Prothioconazole (mit dem chemischen Namen 2-[2-(1-chlorocyclopropyl)-3-(2-chlorophenyl)-2-hydroxpropyl]-1,2-dihydro-3H-1,2,4-triazole-3-thione) (CAS-Nummer 178928-70-6), liegt als Racemat vor. Geeignete Verfahren zu dessen Herstellung werden in der DE-A 195280 beschrieben. Prothioconazole kann in der Thiono-Form der allgemeinen Formel (II) 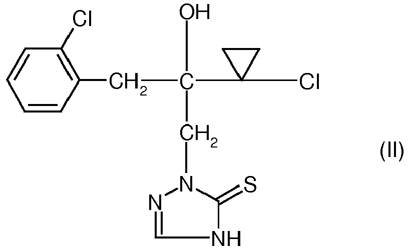 oder in der tautomeren Mercapto-Form der allgemeinen Formel (IIa) 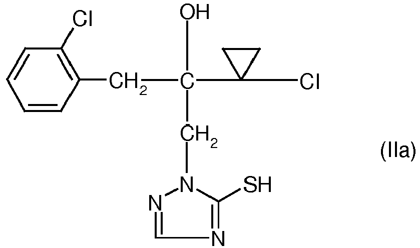 vorliegen. Mit der Verwendung des Begriffs Prothioconazole werden im Folgenden immer die hier dargestellten Isomere sowie weitere mögliche Tautomere abgedeckt.

2-(1-Chlorocyclopropyl)-1-(2-chlorophenyl)-3-(1H-1,2,4-triazol-1-yl)propan-2-ol liegt ebenfalls als Racemat vor und hat die allgemeine Formel (III), wobei wiederum alle tautomeren Formen mit dieser Darstellung umfasst sein sollen.

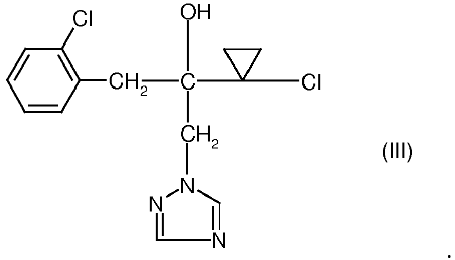

Der Anteil der Komponente a) (Prothioconazole) in den erfindungsgemäßen Formulierungen beträgt vorzugsweise 1 Gew.-% bis 50 Gew.-%, weiter bevorzugt 4 Gew.-% bis 30 Gew.-%, und besonders bevorzugt 5 Gew.-% bis 28 Gew.-% .

Der Anteil der Komponente b) in den erfindungsgemäßen Formulierungen beträgt bevorzugt 1 Gew.-% bis 50 Gew.-%, weiter bevorzugt 2 Gew.-% -45 Gew.-%, noch weiter bevorzugt 5 Gew.-% - 35 Gew.-%, und besonders bevorzugt 8 Gew.-% -25 Gew.-%.

Weiterhin können die Formulierungen gemäß der vorliegenden Erfindung, wie sie vorangehend beschrieben worden sind, jeweils noch weitere Inhaltsstoffe enthalten, wie:
c)
   c1: nicht-ionischer Dispergator/Emulgator,
   c2: ionischer Dispergator/Emulgator
d) andere von a) verschiedene agrochemische Wirkstoffe
e) Lösemittel einsschlieplich OD Carrier
f) Träger (WGs, sowie Aerosile für SC/TK/WG)
g)
   g1: organischer Verdicker
   g2: anorganischer Verdicker
h) weitere Zusatz- und Hilfsstoffe

Formuliertypen werden von der FAO festgelegt und finden sich auf www.fao.org/ag/agp/agpp/pesticid. In der März Ausgabe von 2016 finden sich die üblichen Formuliertypen auf den Seiten 66-231 beschrieben. Bei der erfindungsgemäßen Formulierung handelt es sich um übliche nach FAO beschriebene Formulierungstypen. Beispielhaft seien Suspensionskonzentrate (SC), sowie farbstoffhaltige Konzentrate für die Saatgutbehandlung (FS), Emulsionskonzentrate (EC), wasserdispergierbare Konzentrate (WG), Öldispersionen (OD), Suspoemulsionen (SE), wässrige Emulsionen (EW), Mikroemulsionen (ME) sowie Flüssigformulierungen (SL) hier genannt. Bevorzugt handelt es sich um EC, SC, FS, SE, OD sowie WG Formuliertypen, ganz besonders bevorzugt handelt sich um Formulierungen, wo mindestens ein Wirkstoff nicht gelöst vorliegt. Ganz besonders bevorzugt handelt es sich um FS, SC, SE, OD und WG Formulierungen, ganz besonders bevorzugt um SC, FS und WG Formulierungen.

Weitere Gegenstände der vorliegenden Erfindung sind die Verwendung der erfindungsgemäßen Formulierungen zur Behandlung von Pflanzen sowie entsprechende Verfahren.

In einer bevorzugten Ausführungsform handelt es sich bei IV um kein Lösemittel für Komponente a).

### Nichtionische Emulgier-und Dispergiermittel c1)

Als nichtionische Emulgier-und Dispergiermittel c1) wie Emulgatoren, Netzmittel, Tenside und Dispergatoren kommen übliche, in Formulierungen von agrochemischen Wirkstoffen vorhandene oberflächenative Substanzen in Frage. Beispielhaft genannt seien ethoxylierte Nonylphenole, Umsetzungsprodukte von linearen oder verzweigten Alkoholen mit Ethylenoxid und/oder Propylenoxid, Ethylenoxid-Propylenoxid-Blockcopolymere, Endgruppen-verschlossene und nicht Endgruppen-verschlossene alkoxylierte lineare und verzweigte, gesättigte und ungesättigte Alkohole (z.B. Butoxypolyethylenpropylenglycole), Umsetzungsprodukte von Alkylphenolen mit Ethylenoxid und/oder Propylenoxid, Ethylenoxid- Propylenoxid-Blockcopolymere, Polyethylenglykole und Polypropylenglykole, weiterhin Fettsäureester, Fettsäurepolyglykoletherester, Alkylsulfonate, Alkylsulfate, Arylsulfate, ethoxylierte Arylalkylphenole, wie zum Beispiel Tristyryl-phenol-ethoxylat mit durchschnittlich 16 Ethylenoxid-Einheiten pro Molekül, weiterhin ethoxylierte und propoxylierte Arylalkylphenole sowie sulfatierte oder phosphatierte Arylalkylphenol-ethoxylate bzw. -ethoxy- und -propoxylate. Besonders bevorzugt sind Tristryl-phenol-alkoxylate und Fettsäurepolyglykoletherester. Ganz besonders bevorzugt sind Tristyryl-phenol-ethoxylate, Tristyryl-phenol-ethoxy-propoxylate und Ricinusölpolyglykoletherestern, jeweils einzeln oder in Mischungen. Hinzu kommen gegebenenfalls Additive, wie Tenside oder Ester von Fettsäuren, die zur Verbesserung der biologischen Wirksamkeit beitragen. Geeignete nichtionische Emulgier-und Dispergiermittel c1) sind beispielsweise Soprophor^{®} 796/P, Lucramul^{®} CO30, Lucramul^{®} HOT, Lucramul^{®} PSI 100 oder Synperonic^{®} T304.

Geeignete nicht-ionische Dispergatoren c1) können ebenfalls ausgewählt werden aus der Gruppe enthaltend Polyvinylpyrrolidon (PVP), Polyvinylalkohol, Co-Polymer aus PVP und Dimethylaminoethylmethacrylat, butyliertes PVP, Co-Polymer aus Vinylchlorid und Vinylacetat, und partiell hydrolysiertem Vinylacetat, Phenolharze, modifizierte Cellulose Typen wie beispielsweise Luviskol^{®} (Polyvinylpyrrolidon), Mowiol^{®} (Polyvinylalkohol) oder modifizierte Cellulose. Bevorzugt sind Polyvinylpyrrolidon-Typen, besonders bevorzugt sind Typen von niedrigem Molekulargewicht wie Luviskol^{®} K30 oder Sokalan^{®} K30.

Als weitere nicht-ionische Emulgier-und Dispergiermittel c1) aus der Gruppe der Di- und Triblock-copolymere aus Alkylenoxiden kommen z.B. Verbindungen in Frage, die auf Basis von Ethylen- und Propylenoxid aufgebaut sind, mit mittleren Molmassen zwischen 200 und 10000, bevorzugt 1000 bis 4000 g/mol, wobei der Massenanteil des polyethoxylierte Blocks zwischen 10 und 80% variiert, wie z.B. Synperonic^{®} PE-Reihe (Uniqema), Pluronic^{®} PE-Reihe (BASF), VOP^{®} 32- oder Genapol^{®} PF-Reihe (Clariant).

Der Anteil der nicht-ionischen Emulgier-und Dispergiermittel c1) in den erfindungsgemäßen Suspensionskonzentraten beträgt bevorzugt 1 bis 15 Gew.-%, weiter bevorzugt 2 bis 10 Gew.-%, und besonders bevorzugt 2,5 bis 8 Gew.-%.

### Anionische Emulgier-und Dispergiermittel c2)

Geeignete anionische Emulgier-und Dispergiermittel b1) wie Emulgatoren, Tenside, Netzmittel und Dispergatoren sind beispielweise Alkali-, Erdalkali- oder Ammoniumsalze von Sulfonaten, Sulfaten, Phosphaten, Carboxylaten und deren Mischungen wie z.B. die Salze der Alkylsulphonsäuren oder Alkylphosphorsäuren sowie Alkylarylsulphon- oder Alkylarylphosphorsäuren, Diphenylsulfonate, alpha-Olefinsulfonate, Ligninsulfonate, Sulfonate von Fettsäuren und Ölen, Sulfonate von ethoxylierten Alkylphenolen, Sulfonate von alkoxylierten Arylphenolen, Sulfonate von kondensierten Naphthalinen, Sulfonate von Dodecyl- und Tridecylbenzolen, Sulfonate von Naphthalinen und Alkylnaphthalinen, Sulfosuccinate oder Sulfosuccinamate. Beispiele für Sulfate sind Sulfate von Fettsäuren und Ölen, von ethoxylierten Alkylphenolen, von Alkoholen, von ethoxylierten Alkoholen oder von Fettsäureestern. Beispiele für Phosphate sind Phosphatester. Beispiele für Carboxylate sind Alkylcarboxylate sowie carboxylierte Alkohol- oder Alkylphenolethoxylate. Ebenfalls geeignet ist die Gruppe der anionischen Emulgatoren der Alkalimetall-, Erdalkalimetall- und Ammoniumsalze der Polystyrolsulfonsäuren, Salze der Polyvinylsulphonsäuren, Salze der Alkylnaphthalinsulphonsäuren, Salze von Alkylnaphthalinsulphonsäure-Formaldehyd Kondensationsprodukte, Salze von Kondensationsprodukte der Naphthalinsulphonsäure, Phenolsulphonsäure und Formaldehyd. Beispiele sind Kalziumdodecylbenzensulfonat wie Rhodocal^{®} 70/B (Solvay), Phenylsulfonat CA100 (Clariant) oder Isopropylammoniumdodecylbenzenesulfonate wie Atlox^{®} 3300B (Croda).

Weitere typische Vertreter sind unter anderem Phenylsulfonat CA (Ca-Dodecylbenzolsulfonat), Soprophor^{®}-Typen (gegebenenfalls veresterte Derivate von Tristyrylphenoi-Ethoxylaten), Emmulsogen^{®} 3510 (alkyliertes EO/PO Copolymerisat), Emulsogen^{®} EL 400 (ethoxyliertes Ricinusöl), Tween^{®}-Typen (fettacylierte Sorbitan-Ethoxylate), Calsogen^{®} AR 100 (Ca-Dodecylbenzolsulfonat). Bevorzugt sind Kombinationen aus Salzen alkylierter aromatischer Sulfonsäuren, wie Phenylsulfonat Ca und/oder Calsogen^{®} AR 100, mit alkylierten Copolymerisaten aus Ethylen- und Propylenoxid, wie Emulsogen^{®} 3510. Besonders bevorzugt sind Kombinationen aus Salzen der Dodecylbenzolsulfonsäure, wie Calsogen^{®} AR 1 00 mit alkyliertem Copolymerisat aus Ethylen- und Propylenoxid, wie Emulsogen^{®} 3510.

Beispiele für weitere anionische Emulgier-und Dispergiermittel c2) aus der Gruppe der Naphthalinsulfonate sind Galoryl^{®} MT 800 (Natrium-Dibutylnaphthalinsulfonsäure), Morwet^{®} IP (Natriumdiisopropylnaphthalinsulfonat) und Nekal^{®} BX (Alkylnaphthalinsulfonat). Beispiele für anionische Tenside aus der Gruppe der Kondensationsprodukte von Naphthalinsulfonaten mit Formaldehyd sind Galoryl^{®} DT 201 (Naphthalinsulfonsäure Hydroxypolymer mit Formaldeyd und Methylphenol Natriumsalz), Galoryl^{®} DT 250 (Kondensationsprodukt aus Phenol- und Naphthalinsulfonaten), Reserve^{®} C (Kondensationsprodukt aus Phenol- und Naphthalinsulfonaten) oder Morwet^{®} D-425, Tersperse^{®} 2020. Bevorzugt sind 1,2 mit Di-Butyl- oder Di-Isobutylsubstituierte Naphthalinsulfonate, wie z.B. Produkte wie Galoryl^{®} MT 800 (CFPI-Nufarm) und Nekal^{®} BX (BASF). Weitere typische Tenside sind Soprophor^{®} 3D33, Soprophor^{®} 4D384, Soprophor^{®} BSU, Soprophor^{®} CY/8 (Solvay) und Hoe^{®} S3474 und in Form der Sapogenat^{®} T-Produkte (Clariant), beispielsweise Sapogenat^{®} T 100.

Der Anteil der anionischen Emulgier-und Dispergiermittel c2) in den erfindungsgemäßen Technischen Konzentraten beträgt bevorzugt 2 bis 35 Gew.-%, weiter bevorzugt 3 bis 30 Gew.-%, noch weiter bevorzugt 5 bis 25 Gew.-%, und besonders bevorzugt 10 bis 20 Gew.%.

Der Anteil der anionischen Emulgier-und Dispergiermittel c2) in den erfindungsgemäßen Suspensionskonzentraten beträgt bevorzugt 0,1 bis 10 Gew.-%, weiter bevorzugt 0,2 bis 7 Gew.-%, und besonders bevorzugt 0,3 bis 4 Gew.-%.

### Asrochemische weitere von PTZ verschiedene Wirkstoffe d):

Weitere agrochemische Wirkstoffe d) im Sinne der vorliegenden Erfindung sind fungizide, insektizide oder herbizide Wirkstoffe. In einer alternativen Ausführungsform enthält die erfindungsgemäße Formulierung ein oder mehrere weitere insektizide oder fungizide Wirkstoffe d), besonders bevorzugt ein oder mehrere fungizide Wirkstoffe d). Bevorzugt sind die eingesetzten Wirkstoffe wasserunlöslich.

Eine Übersicht typischer Pflanzenschutzwirkstoffe findet sich im Pesticide manual.

Bevorzugte insektizide Komponenten d) sind beispielsweise Imidacloprid, Nitenpyram, Acetamiprid, Thiacloprid, Thiamethoxam, Clothianidin, Cyantraniliprole, Chlorantraniliprole, Flubendiamide, Tetraniliprole, Cyclaniliprole, Spirodiclofen, Spiromesifen, Spirotetramat, Abamectin, Acrinathrin, Chlorfenapyr, Emamectin, Ethiprole, Fipronil, Flonicamid, Flupyradifurone, Indoxacarb, Metaflumizone, Methoxyfenozid, Milbemycin, Pyridaben, Pyridalyl, Silafluofen, Spinosad, Sulfoxaflor, Triflumuron, die Verbindung aus WO-A 2006/089633 als Beispiel I-1-a-4, die Verbindung offenbart in WO-A 2008/067911 als Beispiel I-1-a-4, die Verbindung offenbart in WO 2013/092350 als Beispiel Ib-14, die Verbindung offenbart in WO 2010/51926 als Beispiel Ik-84.

Bevorzugte fungizide Komponenten d) sind beispielsweise Bixafen, Fenamidone, Fenhexamid, Fluopicolide, Fluopyram, Fluoxastrobin, Iprovalicarb, Isotianil, Isopyrazam, Pencycuron, Penflufen, Propineb, Tebuconazole, Trifloxystrobin, Ametoctradin, Amisulbrom, Azoxystrobin, Benthiavalicarb-isopropyl, Benzovindiflupyr, Boscalid, Carbendazim, Chlorothanonil, Cyazofamid, Cyflufenamid, Cymoxanil, Cyproconazole, Difenoconazole, Ethaboxam, Epoxiconazole, Famoxadone, Fluazinam, Fluquinconazole, Flusilazole, Flutianil, Fluxapyroxad, Isopyrazam, Kresoxim-methyl, Lyserphenvalpyr, Mancozeb, Mandipropamid, Metconazol, Pyriofenone, Folpet, Metaminostrobin, Oxathiapiprolin, Penthiopyrad, Picoxystrobin, Probenazole, Proquinazid, Pydiflumetofen, Pyraclostrobin, Sedaxane, Spiroxamin, Tebufloquin, Tetraconazole, Valiphenalate, Zoxamide, Ziram, N-(5-chloro-2-isopropylbenzyl)-N-cyclopropyl-3-(difluoromethyl)-5-fluoro-1-methyl-1H-pyrazole-4-carboxamide, N-(5-chloro-2-isopropylbenzyl)-N-cyclopropyl-3 -(difluoromethyl)-5 -fluoro-1-methyl-1H-pyrazole-4-carboxamide, 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}phenyl methanesulfonate, 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenylmethane-sulfonate, (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat (Lyserphenvalpyr).

Besonders bevorzugte fungizide Mischungspartner d) des Prothioconazols sind beispielsweise: Tebuconazol, Spiroxamin, Bixafen, Fluoxastrobin, Trifloxystrobin, N-(5-chloro-2-isopropylbenzyl)-N-cyclopropyl-3-(difluoromethyl)-5-fluoro-1-methyl-1H-pyrazole-4-carboxamide, (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat (Lyserphenvalpyr) sowie Fluopyram.

Ganz besonders bevorzugt sind die Mischungen von a) (Prothioconazole) mit ein oder mehreren Verbindungen ausgewählt aus der Gruppe der Verbindungen d):
a) + d) Tebuconazole;
a) + d) Trifloxystrobin;
a) + d) Fluoxastrobin;
a) + d) Bixafen;
a) + d) Fluopyram
a) + d) Spiroxamine;
a) + d) Fluoxastrobin + Trifloxystrobin;
a) + d) Trifloxystrobin+Spiroxamin;
a) + d) Bixafen + Tebuconazole;
a) + d) Bixafen + Fluoxastrobin;
a) + d) Bixafen + Trifloxystrobin;
a) + d) Bixafen + Spiroxamin;
a) + d) Bixafen + Fluopyram;
a) + d) Tebuconazole + Spiroxamin;
a) + d) Tebuconazole + Fluopyram;
a) +d) N-(5-chloro-2-isopropylbenzyl)-N-cyclopropyl-3-(difluoromethyl)-5-fluoro-1-methyl-1 H-pyrazole-4-carboxamide
a) +d) N-(5-chloro-2-isopropylbenzyl)-N-cyclopropyl-3-(difluoromethyl)-5-fluoro-1-methyl-1H-pyrazole-4-carboxamide + Tebuconazole
a) +d) N-(5-chloro-2-isopropylbenzyl)-N-cyclopropyl-3-(difluoromethyl)-5-fluoro-1-methyl-1H-pyrazole-4-carboxamide + Flupyram
a) +d) Bixafen + (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat (Lyserphenvalpyr)

Der Anteil der Komponente d) in den erfindungsgemäßen Formulierungen beträgt vorzugsweise 1 Gew.-% bis 40 Gew.-%, weiter bevorzugt 3 Gew.-% bis 35 Gew.-%.

### Lösemittel e):

Die Formulierungen können Lösemittel e) enthalten, etwa im Falle von EC, OD oder SE Formulierungen.
- Aromatische Kohlenwasserstoffgemische (bevorzugt Naphalin reduziert) wie z.B. Solvesso^{®};
- Aromatische Kohlenwasserstoffe wie z.B. Testbenzin, Petroleum, Alkylbenzole und Spindelöl, Xylol, Toluol oder Alkylnaphthaline;
- Aliphatische/Cyclaliphatische Kohlenwasserstoffe wie z.B. Mineralöle, Hexan, Heptan, Octan, Nonan, Decan, Cylcopentan, Cyclohexan, Decalin oder Weiß-Öl;
- Chlorierte aromatische oder aliphatische Kohlenwasserstoffe wie z.B. Chlorbenzol, Chlorethylen, oder Methylenchlorid, Chloroform oder Tetrachloromethane;
- Alkohole wie z.B. Methanol, Ethanol, iso-Propanol, Butanol, Ethylenglykol, Propylenglykol oder Benzylalkohol;
- Ether wie z.B. Tetrahydrofuran, Tetrahydrofurfurylalkohol, Tetrahydropyran, 1,4-Dioxane, Diethylether, Methyl-tert-butylether, Dihexylether, Dioctylether, Didecylether, Dibenzylether, Dimethylisosorbid, Diphenylether, Ethylphenylether, Phenylbenzylether oder Anisol;
- Monoester/Diester/Glycerin-Ester wie z.B. Ethylacetat, Butylpropionat, PentylPropionat, Benzylacetat, Benzylbenzoat, Butylbenzoat, Rhodiasolv^{®} Polarclean, Rhodiasolv^{®} RPDE, Methyllactat, Ethyllactat, Propyllactat, Butyllactat, 2-Ethylhexyllactat, Ethyl-3-Ethoxypropionat (UCAR^{®} Ester EEP, Dow Chemical Company), Dimethylsuccinat, Diethylsuccinat, Dipropylsuccinat, Dimethyladipat, Diethyladipat, Dipropyladipat, Dimethylglutarat, Diethylglutarat, Dipropylglutarat, Bis(2-ethylhexyl)adipat, Diisopropyladipat, Dimethyl-2-methylglutarat, Dioctylmaleat, Glycerin-monoacetat, Glycerin- diacetat, Glycerin-triacetat, Pflanzenöle wie z.B. Rapsöl, Sonnenblumenöl, Sojabohnenöl, Rizinusöl oder Maisöl;
- Lactone wie z.B. Butyrolacton, alpha-Methyl-gamma-Butyrolacton, gamma-Valerolacton oder delta-Valerolacton;
- Polyethylen/Propylenoxide wie z.B. Monoethylenglycol, Monoethylenglycolmonomethylether, Monoethylenglycol-monoethylether, Monoethylenglycolmonopropylether, Monoethylenglycol-monobutylether, Monoethylenglycolmonopentylether, Monoethylenglycol-monohexylether, Monoethylenglycolmonophenylether, Monoethylenglycol-dimethylether, Monoethylenglycol-diethylether, Monoethylenglycol-dipropylether, Monoethylenglycol-dibutylether, Monoethylenglycoldipentylether, Monoethylenglycol-dihexylether, Monoethylenglycol-diphenylether, sowie deren längere Ethylenglycol Homologen; Monopropylenglycol, Monopropylenglycolmonomethylether, Monopropylenglycol-monoethylether, Monopropylenglycolmonopropylether, Monopropylenglycol-monobutylether, Monopropylenglycolmonopentylether, Monopropylenglycol-monohexylether, Monopropyleneglycolmonophenylether, Monopropylenglycol-dimethylether, Monopropylenglycol-diethylether, Monopropylenglycol-dipropylether, Monopropylenglycol-dibutylether, Monopropylenglycol-dipentylether, Monopropylenglycol-dihexylether, Monopropylenglycol-diphenylether, sowie deren längeren Propylen Homologen; Monoethylenglycol-monomethyletheracetat, Monoethylenglycol-diacetate, sowie deren längere Ethylenglycol Homologen; Monopropylenglycol-monomethyletheracetat, Monopropylenglycoldiacetat, sowie deren längere Polypropylenglycol Homologen;
- Einfache und substituierte Amine wie z.B. Diethylamine, Triethylamine, Diisopropylamine, Diisopropylethylamine, Monoethanolamine, Diethanolamine, Triethanolamine und höher alkoxylierte Amine, Aniline oder Dimethylaniline;
- Amide/Harnstoffe wie z.B. N-Formylmorpholin, N,N-Dimethylformamid, N,N-Dimethylacetamid, N,N-Dimethylbenzamid, N,N-Dimethyloctanamid, N-N-Dimethyldecanamid, N,N-Dimethyl-dec-9-en-l-amid, N,N-Dimethyldodedecanamid, N,N-Dimethyllactamid, N,N-Decylmethylformamid, N-Methyl-2-pyrrolidon, N-Ethyl-2-pyrrolidon, N-Propyl-2-pyrrolidon, N-Butyl-2-pyrrolidon, N-Pentyl-2-pyrrolidon, N-Hexyl-2-pyrrolidon, N-Heptyl-2-pyrrolidon, N-Octyl-2-pyrrolidon, N-Nonyl-2-pyrrolidon, N-Decyl-2-pyrrolidon, N-Undecenyl-2-pyrrolidon, N-Dodecyl-2-pyrrolidon, N-Methyl-2-Piperidon, N-Methyl-caprolactam, N-Octyl-caprolactam, Tetramethylharnstoff, Tetraethylharnstoff, 1,3-Dimethyl-2-imidazolidinon, 1,3,4-Trimethyl-2-imidazolidinon, 1,3-Dimethyl-3,4,5,6-tetrahydro-2(1H)-pyrimidinon, 1-Heptyl-3-methyl-2-Imidazolidinon, 1-Heptyl-1,3-dihydro-3-methyl-2H-Imidazol-2-on;
- Ketone wie z.B. Aceton, Diaceton-alkohol, Methylethylketon, 2-Pentanon, 3-Pentanon, 2-Hexanon, 3-Hexanon, 2-Heptanon, 3-Heptanon, 4-Heptanon, 2-Octanon, 3-Octanon, 4-Octanon, Methylisopropylketon, Methyl-isobutylketone, Methyl-isopentylketon, Ethylisopropylketon, Ethyl-isobutylketon, Ethyl-isopentylketon, Propyl isopropyl keton, Propylisobutylketon, Propyl-isopentylketon, 3,3-Dimethyl-2-Butanon, 2,4-Dimethyl-3-Pentanon, 4,4-Dimethyl-2-Pentanon, 2,6-Dimethyl-4-Heptanon, 2,2,4,4-Tetramethyl-3-Pentanon, Cyclopentanon, Cyclohexanon. Cycloheptanon, Cyclooctanon, 2,4,6-Cycloheptatrien-1-on, Acetophenon, Propiophenon, 1-(4-Methylphenyl)-Ethanon, 1-(4-Ethylphenyl)-Ethanon, 2-Methyl-1-phenyl-1-Propanon, 1-(3-Ethylphenyl)-Ethanon, 4-phenyl-2-Butanon, 1-Phenyl-2-propanon, 1-Phenyl-2-butanon, 2-Phenyl-3-butanon, Butyrophenon oder Valerophenon.
- Nitrile wie z.B. Acetonitril, Propannitril, 2-Methyl-Propannitril, Butannitrile, 3-Methyl-Butannitril, Pentannitril, 4-Methyl-Pentannitril, Hexannitril, Heptannitril, Octannitril, Nonannitril, Decannitril, Benzonnitril, Benzen-acetonitril, Pentan-dinitril, 2-Methyl-pentan-dinitril, Hexan-dinitril, Heptan-dinitril, Octan-dinitril oder Nonan-dinitril;
- Acetale wie z.B. 1,1-Dimethoxymethan; 1,1-Dimethoxyethan; 1,1'-[Methylenbis(oxy)]bis-ethan; 1,1-Diethoxyethan; 1,1'-[Methylenbis(oxy)]bis-propan; 2,4,6,8-Tetraoxanonan, 1,1'-[Methylenebis(oxy)]bis-butan, 2-Methyl-1-[(2-methylpropoxy)methoxy]-propan, 2,4,6,8,10-Pentaoxaundecan, 2,5,7,10-Tetraoxaundecan, 1,3-Dioxolan, 1,3-Dioxan oder 4-Methyl-1,3-Dioxan;Orthoester wie z.B. 1,1,1-Trimethoxymethan, 1,1,1-Trimethoxyethan, 1,1,1-Trimethoxypropan, 2-Methoxy-1,3-dioxolan, 2-Methoxy-2-methyl-1,3-dioxolan, 2-Methoxy-2-methyl-1,3-dioxolan, 2-Ethoxy-1,3-dioxolan, 2-Ethoxy-2-methyl-1,3-dioxolan, 2-Ethyl-2-methoxy-1,3-dioxolan, 2-Methoxy-1,3-dioxan, 2-Methoxy-2-methyl-1,3-dioxan oder 2-Ethoxy-1,3-dioxan;
- Carbonate wie z.B. Dimethylcarbonat, Methylethylcarbonat, Diethylcarbonat, Dipropylcarbonat, Diisopropylcarbonat, Dibutylcarbonat, Dipentylcarbonat, Dihexylcarbonat, Diheptylcarbonat, Dioctylcarbonat, Dinonylcarbonat, Didecylcarbonat, Ethylencarbonat, 4-Methyl-1,3 -Dioxolan-2-on, 4-(Methoxymethyl)-1,3-Dioxolan-2-on, Glycericarbonat, ButylencCarbonat, 4,6-Dimethyl-3-Dioxan-2-onoder Dibenzylcarbonat;
- Phosphate wie z.B. Trimethylphosphat, Triethylphosphat, Tripropylphosphat, Tributylphosphat, Triisobutylphosphat, Tripentylphosphat, Trihexylphosphat, Tris(2-ethylhexyl)phosphat, Tris(2-butoxyethyl) phosphat oder Triphenylphosphat;
- Sulfoxide wie z.B. Dimethylsulfoxid, Diethylsulfoxid, 1,1-Dioxidtetrahydro-thiophen, 1,1-Dioxid- tetrahydro-3-methyl-thiophen oder 1,1-Dioxid-tetrahydro-2,4-dimethyl-thiophe

Des Weiteren können die erfindungsgemäßen Formulierungen gegebenenfalls flüssige Füllstoffe e) wie beispielsweise Pflanzen- oder Mineralöle oder Ester von Pflanzen- oder Mineralölen enthalten. Geeignete Pflanzenöle e) sind alle Öle, die üblicherweise in Agrochemikalien eingesetzt werden können und sich aus Pflanzen gewinnen lassen. Als Beispiele seien Sonnenblumenöl, Rapsöl, Olivenöl, Ricinusöl, Kolzaöl, Maisöl, Baumwollsamenöl, Wallnussöl, Kokosnussöl und Sojaöl genannt. Mögliche Ester sind beispielsweise Ethylhexylpalmitat, Ethylhexyloleat, Ethylhexylmyristat, Ethylhexylcaprylat, Isopropylmyristat, Isopropylpalmitat, Methyloleat, Methylpalmitat, Ethyloleat. Bevorzugt sind Rapsöl-Methylester und Ethylhexylpalmitat. Mögliche Mineralöle sind Exxsol^{®} D100 und Weißöle.

**Tabelle 2: Beispielhafte Handelsnamen und CAS-Nummern bevorzugter Verbindungen e)**

| **Handelsname** | **Firma** | **Allgemeine Beschreibung** | **CAS- Nr.** |
|---|---|---|---|
| Sonnenblumenöl | | Triglyceride von unterschiedlichen C14-C18-Fettsäuren, vorwiegend ungesättigt | 8001-21-6 |
| Rapsöl | | Triglyceride von unterschiedlichen C14-C18-Fettsäuren, vorwiegend ungesättigt | 8002-13-9 |
| Maisöl | | Triglyceride von unterschiedlichen C14-C18-Fettsäuren, vorwiegend ungesättigt | 8001-30-7 |
| Sojaöl | | Triglyceride von unterschiedlichen C14-C18-Fettsäuren, vorwiegend ungesättigt | 8001-22-7 |
| Reisöl | | Triglyceride von unterschiedlichen C14-C18-Fettsäuren, vorwiegend ungesättigt | 68553-81-1 |
| Radia^{®} 7129 | Oleon NV, BE | Ethylhexylpalmitat | 29806-73-3 |
| Crodamol^{®} OP | Croda, UK | | |
| Radia^{®} 7331 | Oleon NV, BE | Ethylhexyloleat | 26399-02-0 |
| Radia^{®} 7128 | Oleon NV, BE | C12/C14-Ethylhexylmyristat/-laurat | 29806-75-5 |
| Radia^{®} 7127 | Oleon NV, BE | Ethylhexyllaurat | 20292-08-4 |
| Radia^{®} 7126 | Oleon NV, BE | C8/10-Ethylhexylcaprylat/caprat | 63321-70-0 |
| Estol^{®} 1514 | Croda | Isopropylmyristat | 110-27-0 |
| Radia^{®} 7104 | Oleon NV, BE | Capryl-,Caprinsäure-Triglyceride, neutrales Pflanzenöl | 73398-61- |
| | | | 5.65381-09-1 |
| Radia^{®} 7732 | Oleon NV, BE | Isopropylpalmitat | 142-91-6 |
| Crodamol^{®} IPM | Croda, UK | | |
| Radia^{®} 7060 | Oleon NV, BE | Methyloleat | 112-62-9 |
| Radia^{®} 7120 | Oleon NV, BE | Methylpalmitat | 112-39-0 |
| Crodamol^{®} EO | Croda | Ethyloleat | 111-62-6 |
| AGNIQUE | Clariant | Rapsöl-Methylester | 67762-38- |
| ME^{®} 18 RD-F, Edenor^{®} MESU | BASF | | 3.85586-25-0 |
| Exxsol^{®} D100 | Exxon Mobil | Hydrotreating-Leichtsieder (Petroleum) | 64742-47-8 |
| Solvesso^{®} 200ND | ExxonMobil | Solvent Naphtha (Petroleum), schwer aromatisch, naphthalinabgereichert | 64742-94-5 |
| Kristol^{®} M14 | Carless | Weißes Mineralöl (Petroleum), C14-C30 verzweigt und linear | 8042-47-5 |
| Marcol^{®} 82 | ExxonMobil | | |
| Ondina^{®} 917 | Shell | | |
| Exxsol^{®}D130 | ExxonMobil | Weißes Mineralöl (Petroleum) | 64742-46-7 |
| Banole^{®} 50 | Total | | |
| Genera^{®}-12 | Total | Weißes Mineralöl (Petroleum) | 72623-86-0 |
| Genera^{®}-9 | Total | Weißes Mineralöl (Petroleum) | 97862-82-3 |

### Trägermaterialien f) für WGs.

Füllstoffe und Trägermaterialien f) in den erfindungsgemäßen Formulierungen sind ausgewählt aus der Gruppe enthaltend Mineralien, Carbonate, Sulfate und Phosphate von Erdalkali- und Erdmetallen, wie Calciumcarbonat, polymere Kohlenhydrate, Gerüstsilikate, wie Fällungskieselsäuren mit geringer Saugfähigkeit und natürliche Gerüstsilikate, wie Kaolin. Typische Vertreter geeigneter Füllstoffe f) sind beispielsweise Agsorb^{®} LVM^{®}-GA (Attapulgit), Harborlite^{®} 300 (Perlit), Collys^{®} HV (modifizierte Stärke), Omya^{®}-Kreide (Calciumcarbonat), Kaolin^{®} Tec 1 (Kaolin, Aluminiumhydrosilicat), Steamic^{®} OOS (Talk, Magnesiumsilicat). Bevorzugt für c2) sind hier natürliche Gerüstsilikate und Calciumcarbonat-Typen wie Omya^{®}-Kreide (Calciumcarbonat), Kaolin Tec 1^{®} (Kaolin) und Harborfite^{®} 300 (Perlit), besonders bevorzugt natürliche Gerüstsilikate wie Kaolin^{®}, Tec^{®} 1 (Kaolin, Aluminiumhydrosilicat) und Harborlite^{®} 300 (Perlit). Ganz besonders bevorzugt werden für WG Formulierungen Kaolin und Calciumcarbonat verwendet. Weitere geeignete Trägermaterialien bzw. Füllstoffe f) sind ausgewählt aus der Gruppe der der hochsaugfähigen Träger mit einer Aufnahmefähigkeit von wenigstens 200 g Dibutylphthalat pro 100 g Trägermaterial. Bevorzugte hochsaugfähige Träger f) sind Kieselsäuren wie beispeilsweise Sipernat^{®}-Typen (synthetische Fällungskieselsäure von hoher Saugfähigkeit) sowie pyrogene Kieselsäure (Aerosil^{®} Typen). Bevorzugt ist Fällungskieselsäure. Der Anteil der Füllstoffe f) in den erfindungsgemäßen TK's beträgt vorzugsweise 0,1 bis 10 Gew.-% , weiter bevorzugt 0,3 bis 8 Gew.-% und ganz besonders bevorzugt 1 bis 7 Gew.-%.

### Verdicker g)

Als Verdicker g) kommen organische Verdicker g1) sowie anorganische Verdicker g2) in Frage.

Organische Verdicker g1) kommen als organisch natürliche bzw. biotechnologisch modifizierte oder organisch synthetische Verdicker in Frage. Typische synthetische Verdicker sind Rheostrux^{®} (Croda), Thixin^{®}- oder Thixatrol^{®}-Reihe (Elementis). Diese sind typischerweise auf Basis von Acrylaten. Typische organische Verdicker sind auf Basis von Xanthan oder Cellulose (wie etwa Hydroxyethyl oder Carboxymethylcellulose) oder einer Kombination davon. Weitere typische Vertreter sind auf Basis von Lignin (wie Ligninsulfonate, Borresperse^{®}NA, REAX^{®} 88 oder Kraftsperse 25 S). Bevorzugt werden natürliche modifzierte Verdicker auf Basis von Xanthan verwendet. Typische Vertreter sind beispielsweise Rhodopol^{®} (Solvay) und Kelzan^{®} (Kelco Corp.) sowie Satiaxane^{®} (Cargill).

Der Anteil der organischen Verdicker g1) in den erfindungsgemäßen SC's B) beträgt kleiner gleich 5 Gew.-%, bevorzugt 0,01 bis 1,0 Gew.-%, besonders bevorzugt 0,01 bis 0,6 Gew.-%, ganz besonders bevorzugt 0,05 bis 0,5 Gew.-% und ganz besonders bevorzugt 0,1 bis 0,3 Gew.-%.

Als anorganische Verdicker g2) geeignet sind beispielsweise modifizierte natürliche Silikate, wie chemisch modifizierte Bentonite, Hectorite, Attapulgite, Montmorillonite, Smektite oder andere Silikatmineralien, wie Bentone^{®} (Elementis), Attagel^{®} (Engelhard), Agsorb^{®} (Oil-Dri Corporation) oder Hectorite^{®} (Akzo Nobel) oder der Van Gel-Reihe (R.T. Vanderbilt).

Der Anteil anorganischer Verdicker g2) in den erfindungsgemäßen Formulierungen beträgt bis 0 bis 5 Gew.-%, bevorzugt 0,1 bis 3 Gew.-%, besonders bevorzugt 0,2 bis 1,5 Gew.-%, ganz besonders bevorzugt 0,3 bis 1,5 Gew.-% und ganz besonders bevorzugt 0,4 bis 1,3 Gew.-%.. Bevorzugt wird eine Mischung aus Verdicker g1) und g2) eingesetzt. Besonders bevorzugt werden im Falle von SCs ausschließlich organische Verdicker d1) eingesetzt. Ganz besonders bevorzugt sind solche Verdicker g1) auf Basis von Xanthan (wie Rhodopol^{®} G von Solvay).

### Weitere Komponenten h)

Darüber hinaus können die erfindungsgemäßen SC's oder TK's oder WG's als weitere Komponenten h) gegebenenfalls noch enthalten:
Netzmittel, pH-Wert-Einstellungsmittel, Entschäumer, Biozide, Desintergrationsmittel, Haftvermittler, Frostschutzmittel, Konservierungsmittel, Farbstoffe oder Dünger, sowie von Komponente c) verschiedene Tenside.

Geeignete Entschäumer sind oberflächenaktive Verbindungen auf Silikon- bzw. Silanbasis wie die Tegopren^{®}-Produkte (Goldschmidt), die SE^{®}-Produkte (Wacker), sowie die Bevaloid^{®}- (Kemira), Rhodorsil^{®}- (Solvay) und Silcolapse^{®}-Produkte (Blustar Silicones) bevorzugt sind SE^{®}- (Wacker), Rhodorsil^{®}-und Silcolapse^{®}-Produkte, besonders bevorzugt sind z.B. Produkte wie Silcolapse^{®} 5020.

Geeignete Frostschutzmittel sind solche aus der Gruppe der Harnstoffe, Diole und Polyole, wie Ethylenglycol und Propylenglycol, Glycerin bevorzugt Propylenglycol oder Glycerin.

Geeignete Konservierungsmittel sind z.B. Produkte wie Acticide^{®} MBS (Biozid, Thor Chemie), CIT, MIT oder BIT wie etwa Proxel^{®} GXL (BIT), Acticide^{®} SPX (MIT, CIT).

Geeignete Haftvermittler können ausgewählt werden aus der Gruppe Polyvinylpyrrolidon (PVP), Polyvinylalkohol, Co-Polymer aus PVP und Dimethylaminoethylmethacrylat, butyliertes PVP, Co-Polymer aus Vinylchlorid und Vinylacetat, Na-Salz des Co-Polymers aus Propensultansäure und partiell hydrolysiertem Vinylacetat, Natriumcaseinat, Phenolharze, modifizierte CelluloseTypen wie beispielsweise Luviskol^{®} (Polyvinylpyrrolidon), Mowiol^{®} (Polyvinylalkohol), modifizierte Cellulose

Geeignete Antischaum-Mittel können ausgewählt werden aus der Gruppe der Ester der Phosphorsäure mit niederen Alkoholen, C₆-C₁₀ Alkohole, Silicontenside (Suspoemulsionen von hydrophobisierten Kieselsäurepartikeln in wässrigen Emulsionskonzentraten auf der Basis flüssiger Silicontenside), wie Polydimethylsiloxan, sowie deren Absorbate an festes Trägermaterial wie beispielsweise Rhodorsil^{®} 432 (Silicontensid), Butylphosphat, iso-Butylphosphat, n-Octanol, Wacker ASP15 (Polydimethylsiloxan, an festem Träger absorbiert), Antischaum-Mittel^{®}SE (Polydimethylsiloxan). Bevorzugt sind Suspoemulsionen von hydrophobisierten Kieselsäurepartikeln in wässrigen Emulsionskonzentraten auf der Basis flüssiger Silicontenside, wie Antischaum-Mittel^{®} SE (Polydimethylsiloxan), und feste Antischaum-Mittel, wie Wacker ASP 15 (Polydimethylsiloxan).

Weitere, gegebenenfalls in den erfindungsgemäßen Formulierungen enthaltene Zusätze h) sind Penetrationsförderer, Netzmittel, Spreitmittel und/oder Retentionsmittel. Geeignet sind alle Substanzen, die üblicherweise in Agrochemikalien zu diesem Zweck eingesetzt werden können.

Als Zusätze h) geeignet sind beispielsweise
- ethoxylierte verzweigte Alkohole (z.B. Genapol^{®} Typ X) mit 2-20 EO-Einheiten;
- ethoxylierte verzweigte Alkohole mit endständigem Methyl (z.B. Genapol^{®} Typ XM) mit 2-20 EO-Einheiten;
- ethoxylierte Kokosnussalkohole (z.B. Genapol^{®} C-Typen) mit 2-20 EO-Einheiten;
- ethoxylierte C12/15-Alkohole (z.B. Synperonic^{®} A-Typen) mit 2-20 EO-Einheiten;
- propoxy-ethoxylierte Alkohole, verzweigt oder linear, z.B. Antarox^{®} B/848, Atlas^{®} G5000, Lucramul^{®} HOT 5902;
- propoxy-ethoxylierte Fettsäuren mit endständigem Methyl, z.B. Leofat^{®} OC0503M;
- organomodifizierte Polysiloxane, z.B. BreakThru^{®} OE444, BreakThru^{®} S240, Silwett^{®} L77, Silwett^{®} 408;
- Mono- und Diester von Sulfosuccinat-Na-Salzen mit verzweigten oder linearen Alkoholen mit 1-10 Kohlenstoffatomen;
- ethoxylierte Diacetylendiole (z.B. Surfynol^{®}).

**Tabelle 3: Beispielhafte Handelsnamen und CAS-Nummern bevorzugter Verbindungen h)**

| **Handelsname** | **Firma** | **Allgemeine Beschreibung** | **CAS- Nr.** |
|---|---|---|---|
| Lucramul^{®} HOT 5902 | Levaco | Alkoholethoxylat-propoxylat (C8-P08/E06) | 64366-70-7 |
| Genapol^{®} X060 | Clariant | Alkoholethoxylat (iso-C13-EO6) | 9043-30-5 |
| Genapol^{®} XM 060 | Clariant | Alkoholethoxylat (iso-C13-EO6/mit Me-Endgruppe) | 345642-79-7 |
| Triton^{®} GR 7 ME | Dow | Dioctylsulfosuccinat, Natriumsalz | 577-11-7 |
| BreakThru^{®} OE 444 | Evonik Industries | Siloxane und Silicone, Cetyl-Me, Di-Me | 191044-49-2 |
| BreakThru^{®} S240 | Evonik Industries | Polyethermodifiziertes Trisiloxan | 134180-76-0 |
| Silwett^{®} L77 | Momentive | Polyalkylenoxidmodifiziertes Heptamethyltrisiloxan | 67674-67-3 |
| Silwett^{®} 408 | Momentive | Polyalkylenoxidmodifiziertes Heptamethyltrisiloxan | 67674-67-3 |
| Antarox^{®} B/848 | Solvay | Oxiran, Methyl-, Polymer mit Oxiran, Monobutylether | 9038-95-3 |
| Atlas^{®} G5000 | Croda | Oxiran, Methyl-, Polymer mit Oxiran, Monobutylether | 9038-95-3 |
| Leofat^{®} OC-0503M | Lion Chemical, JP | Oxiran, Methyl-, Polymer mit Oxiran, Mono-(9Z)-9-octadecenoat, Methylether, Block | 181141-31-1 |
| Surfynol^{®} 440 | Air Products | 2.4.7.9-Tetramethyldec-5-in-4.7-diol, ethoxyliert | 9014-85-1 |

Als Entschäumer h sind alle Substanzen geeignet, die üblicherweise in Agrochemikalien zu diesem Zweck eingesetzt werden können. Bevorzugt sind Siliconöle, Siliconölzubereitungen Magnesiumstearat, Phosphin- und Phosphonsäuren. Beispiele sind Silcolapse^{®} 482 von Bluestar Silicones, Silfoam^{®} SC1132 von Wacker [Dimethylsiloxane und -silicone, CAS-Nr. 63148-62-9], SAG 1538 oder SAG 1572 von Momentive [Dimethylsiloxane und -silicone, CAS-Nr. 63148-62-9] oder Fluowet^{®} PL 80.

Mögliche Konservierungsmittel h sind alle Substanzen, die üblicherweise in Agrochemikalien zu diesem Zweck eingesetzt werden können. Als Konservierungsmittel eignen sich beispielsweise Zubereitungen mit 5-Chlor-2-methyl-4-isothiazolin-3-on [CIT; CAS-Nr. 26172-55-4], 2-Methyl-4-isothiazolin-3-on [MIT, CAS-Nr. 2682-20-4] oder 1.2-Benzisothiazol-3(2H)-on [BIT, CAS-Nr. 2634-33-5]. Als Beispiele seien Preventol^{®} D7 (Lanxess), Kathon^{®} CG/ICP (Rohm & Haas), Acticide^{®} SPX (Thor GmbH) und Proxel^{®} GXL (Arch Chemicals) genannt.

Als Antioxidantien h sind alle Substanzen geeignet, die üblicherweise in Agrochemikalien zu diesem Zweck eingesetzt werden können. Bevorzugt ist Butylhydroxytoluol [3.5-Di-tert.-butyl-4-hydroxytoluol, CAS-Nr. 128-37-0] und Zitronensäure.

Mögliche Farbstoffe h sind alle Substanzen, die üblicherweise in Agrochemikalien zu diesem Zweck eingesetzt werden können. Als Beispiele seien Titandioxid, Ruß, Zinkoxid, blaue Pigmente, rote Pigmente und Permanent Red FGR genannt.

Als inerte Füllstoffe h) sind alle Substanzen geeignet, die üblicherweise in Agrochemikalien zu diesem Zweck eingesetzt werden können und die nicht als Verdicker fungieren. Bevorzugt sind anorganische Partikel, wie Carbonate, Silicate und Oxide, und auch organische Substanzen, wie Harnstoff-Formaldehyd-Kondensate. Als Beispiele seien Kaolin, Rutil, Siliciumdioxid ("hochdisperse Kieselsäure"), Kieselgel sowie natürliche und synthetische Silicate, zudem Talk genannt.

Weiterhin Gegenstand der vorliegenden Erfindung sind wasserdispergierbare Technische Konzentrate (TK's) basierend auf Claim 1 enthaltend,
- ein oder mehrere anionische Emulgatoren c2,
- ein oder mehrere nichtionische Emulgatoren c1),
- mindestens ein oder mehrere Trägermaterialien f,

Ebenfalls Gegenstand der vorliegenden Erfindung sind Suspensionskonzentrate (SC's) enthaltend
- ein oder mehrere anionische Emulgatoren c2,
- ein oder mehrere nichtionische Emulgatoren c1,
- mindestens ein oder mehrere Verdicker g).

Ebenfalls Gegenstand der vorliegenden Erfindung sind EC's enthaltend
- ein oder mehrere nichtionische Emulgatoren c1,
- mindestens ein Lösemittel e)

Ebenfalls Gegenstand der vorliegenden Erfindung sind EC's enthaltend
- ein oder mehrere nichtionische Emulgatoren c1,
- mindestens ein weiterer agrochemischer Wirkstoff, wobei dieser bevorzugt ein Fungizid ist.

Ebenfalls Gegenstand der vorliegenden Erfindung sind OD's enthaltend
- ein oder mehrere nichtionische Emulgatoren c1,
- ein oder mehrere anionische Emulgatoren c2
- mindestens ein Lösemittel e)
- mindestens einen Verdicker g)

### Applikation

Als Applikationsformen können alle, dem Fachmann als gebräuchlich bekannten Verfahren verwendet werden; beispielsweise genannt seien: Spritzen, Tauchen, Nebeln sowie eine Reihe spezieller Verfahren zur direkten unter- oder oberirdischen Behandlung von gesamten Pflanzen oder Teilen (Saatgut, Wurzel, Stolonen, Stängel, Stamm, Blatt), wie beispielsweise Stamminjektion bei Bäumen oder Stängelbandagen bei perennierenden Pflanzen, und eine Reihe spezieller indirekter Applikationsverfahren.

Die jeweilige flächen- und/oder objektbezogene Aufwandmenge der Pflanzenschutzmittel unterschiedlichster Formulierungstypen zur Bekämpfung der genannten Schadorganismen variiert sehr stark. Im Allgemeinen werden hierfür die, dem Fachmann als gebräuchlich für das jeweilige Einsatzgebiet bekannten Applikationsmedien in den gebräuchlichen Mengen eingesetzt; wie beispielsweise von mehreren hundert Liter Wasser pro Hektar bei Standard-Spritzverfahren über wenige Liter Öl pro Hektar bei der 'Ultra Low Volume'-Flugzeugapplikation bis hin zu wenigen Millilitern einer physiologischen Lösungen bei Injektionsverfahren. Die Konzentrationen der erfindungsgemäßen Pflanzenschutzmittel in den entsprechenden Applikationsmedien variieren daher in einem weiten Bereich und sind vom jeweiligen Einsatzgebiet abhängig. Im Allgemeinen werden Konzentrationen verwendet, die dem Fachmann als gebräuchlich für das jeweilige Einsatzgebiet bekannt sind. Bevorzugt sind Konzentrationen von 0,01 Gew.% bis 99 Gew.%, besonders bevorzugt von 0,1 Gew.% bis 90 Gew.%.

Die erfindungsgemäßen agrochemischen Formulierungen können z.B. in den für Flüssigpräparate üblichen Zubereitungsformen entweder als solche oder nach vorherigem Verdünnen mit Wasser ausgebracht werden, also z.B. als Emulsionen, Suspensionen oder Lösungen. Die Anwendung erfolgt dabei nach üblichen Methoden, also z.B. durch Verspritzen, Gießen oder Injizieren.

In Abhängigkeit von der Art des neben Prothioconazole möglicherweise zusätzlich vorliegenden Wirkstoffs sind die erfindungsgemäßen Formulierungen zur Bekämpfung einer großen Zahl von Schädlingen nützlich und können sowohl zur Behandlung von Pflanzenkulturen, aber auch von unbelebter Materie und im Haushalt eingesetzt werden.

Unter "Schädlingen" oder "schädlichen Organismen" werden hier alle Arten von Schädlingen verstanden, die mit organischen Pflanzenschutzwirkstoffen, d.h. Pflanzenschutzmitteln, insbesondere Fungiziden und Mischungen von Fungiziden mit anderen Pflanzenschutzmitteln, bekämpft bzw. unter Kontrolle behalten werden können. Der Begriff Schädling umfasst daher pflanzenschädigende Organismen, insbesondere Schadpilze und deren Sporen, aber auch schädliche Insekten, Arachniden, Nematoden und Schadpflanzen. Der Begriff "Kontrolle" umfasst sowohl die kurative Behandlung, d.h. die Behandlung von befallenen Pflanzen mit einer erfindungsgemäßen Formulierung, als auch die protektive Behandlung, d.h. die Behandlung von Pflanzen zum Schutz vor einem Schädlingsbefall.

Die vorliegende Erfindung betrifft somit auch die Verwendung von hierin beschriebenen Formulierungen zur Bekämpfung von Schädlingen, insbesondere Pflanzenschädlingen; und ein Verfahren zur Bekämpfung von schädlichen Organismen, insbesondere von pflanzenschädigenden Organismen, umfassend das in Kontakt Bringen der schädlichen Organismen, ihres Habitats, ihrer Wirte, wie Pflanzen und Saatgut, sowie des Erdreichs, des Gebiets und der Umgebung in denen sie wachsen oder wachsen könnten, aber auch von Materialien, Pflanzen, Saatgut, Erdreich, Oberflächen oder Räumen, die vor dem Angriff oder dem Befall durch pflanzenschädigende Organismen geschützt werden sollen, mit einer wirksamen Menge der erfindungsgemäßen Formulierungen.

Ein weiterer Gesichtspunkt der Erfindung betrifft die Verwendung der hierin beschriebenen Formulierungen zum Schutz von Pflanzen einschließlich Saatgut, insbesondere um Nutzpflanzen vor dem Befall durch schädliche Organismen, insbesondere Schadpilzen, zu schützen. Die vorliegende Erfindung betrifft somit auch die Verwendung der Formulierungen zur Bekämpfung pflanzenschädlicher Organismen wie beispielsweise Schadpilzen, Insekten, Arachniden, Nematoden und Schadpflanzen, insbesondere zur Bekämpfung von Schadpilzen.

Die Formulierungen der Erfindung können im Pflanzenschutz vor allem als Blatt-, Beiz- und Bodenfungizide in an sich bekannter Weise zur Bekämpfung von pflanzenpathogenen Pilzen eingesetzt werden.

Als Pflanzen, welche mit den erfindungsgemäßen Formulierungen behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp*., *Juglandaceae sp*., *Betulaceae sp*., *Anacardiaceae sp*., *Fagaceae sp*., *Moraceae sp*., *Oleaceae sp*., *Actinidaceae sp*., *Lauraceae sp*., *Musaceae sp.* (beispielsweise Bananenbäume und -plantagen), *Rubiaceae sp.* (beispielsweise Kaffee), *Theaceae sp., Sterculiceae sp., Rutaceae sp.* (beispielsweise Zitronen, Organen und Grapefruit); *Solanaceae sp.* (beispielsweise Tomaten), *Liliaceae sp., Asteraceae sp.* (beispielsweise Salat), *Umbelliferae sp., Cruciferae sp., Chenopodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Asteraceae sp.* (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp.* (beispielsweise Sojabohne), *Solanaceae sp.* (beispielsweise Kartoffeln), *Chenopodiaceae sp.* (beispielsweise Zuckerrübe, Futterrübe, Mangold, Rote Rübe); Zuckerrohr, Mohn, Olive, Kokosnuss, Kakao, Tabak und Nutzpflanzen und Zierpflanzen in Garten und Wald; sowie jeweils genetisch modifizierte Arten dieser Pflanzen, sowie die Samen dieser Pflanzen.

Bevorzugt werden die erfindungsgemäßen Formulierungen zur Behandlung von Weizen, Gerste, Roggen, Soja, Zwiebeln, Mais und Erdnüssen eingesetzt.

Insbesondere lassen sich mit den erfindungsmäßigen Formulierungen des Prothioconazols grundsätzlich alle Schadpilzerkrankungen bekämpfen, die auch mit den bekannten Formulierungen des Prothioconazols bekämpft werden können. Abhängig von dem jeweiligen, gegebenenfalls vorliegenden Mischungspartner handelt es sich beispielsweise um die folgenden Pflanzenerkrankungen:
*Alternaria* Arten an Gemüse, Raps, Zuckerrüben, Soja, Getreide, Baumwolle, Obst und Reis (z.B. *A. solani* oder *A. alternata* an Kartoffel und anderen Pflanzen), *Aphanomyces* Arten an Zuckerrüben und Gemüse, *Ascochyta sp.* an Baumwolle und Reis, *Bipalaris-* und *Drechslera* Arten an Mais, Getreide, Reis und Rasen (z.B. *teres* an Gerste, *D. tritci-repentis* an Weizen), *Blumeria graminis* (Echter Mehltau) an Getreide, *Botrytis cinerea* (Grauschimmel) an Erdbeeren, Gemüse, Blumen und Weinreben, *Botryodiplodia sp.* an Baumwolle, *Bremia lactucae* an Salat, *Cerospora* Arten an Mais, Sojabohnen, Reis und Zuckerrüben (z.B. *C. beticula* an Zuckerrüben), *Cochliobolus* Arten an Mais, Getreide, Reis (z.B. *Cochliobolus sativus* an Getreide, *Cochliobolus miyabeanus* an Reis), *Corynespora sp.* an Sojabohnen, Baumwolle und anderen Pflanzen, *Colletotrichum* Arten an Sojabohnen, Baumwolle und anderen Pflanzen (z.B. *C. acutatum* an verschiedenen Pflanzen), *Curvularia sp.* an Getreide und Reis, *Diplodia sp.* an Getreide und Reis, *Exserohilum* Arten an Mais, *Erysiphe cichoracearum* und *Sphaerotheca fuliginea* an Gurkengewächsen, *Fusarium* und *Verticillium* Arten (z.B. *V. dahliae)* an verschiedenen Pflanzen (z.B. *F. graminearum* an Weizen), *Gaeumanomyces graminis* an Getreide, *Gibberella* Arten an Getreide und Reis (z.B. *Gibberella fujikuroi* an Reis), *Grainstaining complex* an Reis, *Helminthosporium* Arten (z.B. *H. graminicola*) an Mais und Reis, *Macrophomina sp.* an Soja und Baumwolle, *Michrodochium sp.* (z.B. *M. nivale* an Getreide), *Mycosphaerella* Arten an Getreide, Bananen und Erdnüssen (*M. graminicola* an Weizen, *M. fijiesis* an Banane), *Phaeoisaripsis sp.* an Sojabohnen, *Phakopsara sp.* (z.B. *P. pachyrhizi* und *P. meibomiae* an Sojabohnen), *Phoma sp.* an Soja, *Phomopsis* Arten an Sojabohnen, Sonnenblumen und Weinreben (*P. viticola* an Weinreben, *P. helianthii* an Sonnenblumen), *Phytophthora infestans* an Kartoffeln und Tomaten, *Plasmopara viticola* an Weinreben, *Penecilium sp.* an Soja und Baumwolle, *Podosphaera leucotricha* an Apfel, *Pseudocercosporella herpotrichoides* an Getreide, *Pseudoperonospora* Arten an Hopfen und Gurkengewächsen (z.B. *P. cubenis* an Gurke), *Puccinia* Arten an Getreide, Mais und Spargel (P. *triticina* und *P. striformis* an Weizen, *P. asparagi* an Spargel), *Pyrenophora* Arten an Getreide, *Pyricularia oryzae, Corticium sasakii, Sarocladium oryzae, S. attenuatum, Entyloma oryzae* an Reis, *Pyricularia grisea* an Rasen und Getreide, *Pythium spp.* an Rasen, Reis, Mais, Baumwolle, Raps, Sonnenblumen, Zuckerrüben, Gemüse und anderen Pflanzen, *Rhizoctonia*-Arten (z.B. *R. solani*) an Baumwolle, Reis, Kartoffeln, Rasen, Mais, Raps, Kartoffeln, Zuckerrüben, Gemüse und anderen Pflanzen, *Rynchosporium sp.* (z.B. *R. secalis*) an Reis und Getreide, *Sclerotinia* Arten (z.B. S. *sclerotiorum*) an Raps, Sonnenblumen und anderen Pflanzen, *Septoria tritici* und *Stagonospora nodorum* an Weizen, *Erysiphe* (syn. *Uncinulanecator*) an Weinrebe, *Setospaeria* Arten an Mais und Rasen, *Sphacelotheca reilinia* an Mais, *Thievaliopsis* Arten an Sojabohnen und Baumwolle, *Tilletia* Arten an Getreide, *Ustilago* Arten an Getreide, Mais und Zuckerrübe, und *Venturia* Arten (Schorf) an Apfel und Birne (z.B. V. *inaequalis* an Apfel).

Die erfindungsgemäßen Formulierungen können unverdünnt oder mit Wasser verdünnt appliziert werden. In der Regel werden sie mit wenigstens einem Teil Wasser, bevorzugt mit 10 Teilen Wasser und besonders bevorzugt mit wenigstens 100 Teilen Wasser, beispielsweise mit 1 bis 10.000, vorzugsweise 10 bis 5.000 und ganz besonders bevorzugt mit 50 bis 24000 Teilen Wasser bezüglich eines Teiles der Formulierung verdünnt.

Ebenfalls Gegenstand der vorliegenden Erfindung ist eine Emulsion erhältlich durch Mischen von Wasser mit der erfindungsgemäßen flüssigen Formulierung. Das Mischungsverhältnis von Wasser zu Emulsionskonzentrat kann im Bereich von 1000 zu 1 bis 1 zu 1, bevorzugt 400 zu 1 bis 10 zu 1 betragen.

Die Verdünnung wird erreicht, indem die erfindungsgemäßen Emulsionskonzentrate zu dem Wasser gegossen werden. Zur raschen Vermischung des Konzentrats mit Wasser wird üblicherweise Agitation, wie beispielsweise Rühren, eingesetzt. Allerdings ist eine Agitation in der Regel nicht notwendig. Obwohl die Temperatur für den Verdünnungsvorgang ein unkritischer Faktor ist, werden Verdünnungen üblicherweise bei Temperaturen im Bereich von 0°C bis 50°C, insbesondere bei 10°C bis 30°C oder bei Umgebungstemperatur durchgeführt.

Das zum Verdünnen eingesetzte Wasser ist in der Regel Leitungswasser. Das Wasser kann aber bereits wasserlösliche oder feindispergierte Verbindungen beinhalten, die im Pflanzenschutz verwendet werden, wie etwa Nährstoffe, Düngemittel oder Pestizide.

Zu der erfindungsgemäßen Emulsion können als Vormischung oder gegebenenfalls erst kurz vor der Verwendung (Tank-Mix) verschiedene Arten von Ölen, Netzmitteln, Adjuvantien, Düngemitteln oder Mikronährstoffen sowie weitere Pestizide (z.B. Herbizide, Insektizide, Fungizide, Wachstumsregulatoren, Safener) hinzugefügt werden. Diese Mittel können den erfindungsgemäßen Formulierungen im Gewichtsverhältnis 1:100 bis 100:1,bevorzugt 1:10 bis 10:1,zugemischt werden.

Der Verwender appliziert die erfindungsgemäße Formulierung üblicherweise aus einem Vordosierungsgerät, einer Rückenspritze, einem Spritztank, einem Spritzflugzeug oder einem Bewässerungssystem. üblicherweise wird die erfindungsgemäße Formulierung mit Wasser, Puffer und/oder weiteren Hilfsstoffen auf die gewünschte Ausbringungskonzentration verdünnt, wodurch man die gebrauchsfertige Spritzflüssigkeit bzw. agrochemische Zusammensetzung der Erfindung erhält. Üblicherweise werden pro Hektar landwirtschaftlicher Nutzflache 20 bis 2000 Liter, bevorzugt 50 bis 400 Liter, der gebrauchsfertigen Spritzbrühe ausgebracht.

Die erforderlichen Aufwandmengen der reinen Wirkstoffe ohne Formulierungshilfsstoffe hängen von der Intensität des Schädlingsbefalls, von der Entwicklungsphase der Pflanzen, von den klimatischen Bedingungen des Einsatzortes und dem Applikationsverfahren ab. Im Allgemeinen liegt die Aufwandmenge im Bereich von 0,001 bis 3 kg, bevorzugt von 0,005 bis 2 kg, besonders bevorzugt von 0,01 bis 1 kg und ganz besonders bevorzugt von 50 bis 500 g Wirkstoff pro Hektar, wobei Wirkstoff hier Prothioconazole zuzüglich möglicher weiterer Wirkstoffe bedeutet.

Die in der Regel verdünnten Formulierungen der Erfindung werden hauptsächlich durch Besprühen, insbesondere Besprühen der Blätter, appliziert. Die Applikation kann mit dem Fachmann bekannten Sprühtechniken, beispielsweise unter Verwendung von Wasser als Träger und Spritzbrühenmengen von etwa 50 bis 1 000 Litern pro Hektar, beispielsweise von 100 bis 00 Litern pro Hektar, durchgeführt werden.

Die neuen Prothioconazole-haltigen Formulierungen verfügen über vorteilhafte Eigenschaften für die Behandlung von Pflanzen, insbesondere zeichnen sie sich durch gute Anwendungseigenschaften, hohe Stabilität und hohe fungizide Aktivität aus.

Die Erfindung wird durch die Beispiele näher erläutert ohne sie darauf zu beschränken.

### Beispiele

### Verwendete Einsatzstoffe:

Die in den nachfolgenden Beispielen verwendeten Begriffe haben folgende Bedeutung:

| | |
|---|---|
| Prothioconazol | PTZ (Bayer AG) |
| Synperonic F127 | Propylenoxid-Ethylenoxid-(PO-EO)-Blockpolymer (Croda) |
| Soprophor 4D384 | Tristyrylphenol ethoxylate sulfate-ammonium salt (Solvay) |
| Citronensäure | mehrbasige organische Säure |
| Rhodopol^{®} 23 | Xanthan Derivat (Solvay) |
| Silcolapse^{®} 411 | Silikon-Entschäumer (Solvay) |
| Silfoam^{®} SE 2 | Silikon-Entschäumer (Wacker) |
| Glycerin | Frostschutzmittel |
| Proxel^{®} GXL | Konservierungsmittel (Biozid, Proxel) |
| Kaolin TEC1 | Aluminiumhydrosilicat |
| Sipernat^{®} 22S | Fällungskieselsäure |
| Sokalan^{®} K30 | Polyvinylpyrollidon, nicht-ionisches Dispergiermittel |
| Solvesso 200 ND | Mineralöl, ExxonMobil, Naphthalinfrei |
| Calsogen^{®} 4814 | Calcium-dodecylbenzolsulphonate in 2-Ethylhexanol (ca. 40%), Clariant |
| Emulsogen^{®} 3510 | nichtionisches Tensid, n-butyl-PO-EO block oxalkylate, Clariant |
| Lucramul® CO30 | Nichtionischer Emulgator, Rizinusöl ethoxyliert, Levaco Chemicals, DE |
| Rhodocal 60BE | Calcium-dodecylbenzenesulphonate in 2-Ethylhexanol (ca.40%), Solvay |
| EL400 | Ethoxyliertes (40EO) Rizinusöl, Croda |
| Aerosil 972 | hydrophobe disperse Kieselsäure, Evonik |
| Soprophor^{®} 796/P | Nichtionischer Emulgator, Ethoxyliertes-Propoxyliertes Tristyrylphenol |
| | Solvay |
| SAG1572 | Wässrige Emulsion von Polydimethylsiloxan Momentive® |
| Hallcomid® 1025 | Di-Methyl-Decenamid Stepan |
| Genagen® 4296 | Di-Methyl-Decanamid Clariant |
| Agnique® KE 3308 | Di-Methyl-Decanamid BASF |
| 9-Decensäure | Sigma-Aldrich (CAS-Nummer: 14436-32-9) |
| Limonen | Sigma-Aldrich, erhältlich in verschieden Isomeren unter den folgenden |

CAS-Nummern 5989-27-5 [(D)-(+)-Limonen], 5989-54-8 [(S)-(-)-Limonen], 138-86-3 [Dipenten unspezifiziert], 7705-14-8 [(±)-Limonen], 6876-12-6 [trans-1-Methyl-4-(methylvinyl)cyclohexen

### Allgemeine Herstellung eines wässrigen Suspensionskonzentrats (SC):

Es wird zunächst Wasser bei Raumtemperatur vorgelegt. Unter Rühren werden die Wirkstoffe sowie die weiteren Komponenten hinzugegeben (ohne besondere Reihenfolge). Es erfolgt eine Vorzerkleinerung mit einer Kolloidmühle, gefolgt von einer Nassvermahlung beispielsweise mittels einer Perlmühle. Abschließend wird der organische Verdicker zugegeben.

### Allgemeine Herstellung eines Wasserdispergierbaren Granulat (WG)

Es wird Wasser bei Raumtemperatur vorgelegt. Unter Rühren werden die Wirkstoffe sowie die weiteren Komponenten hinzugegeben (ohne besondere Reihenfolge). Es erfolgt eine Vorzerkleinerung mit einer Kolloidmühle, gefolgt von einer Nassvermahlung beispielsweise mittels einer Perlmühle. Die so erhaltenen erfindungsgemäßen TK Slurries werden zum WG weiterverarbeitet. Es erfolgt eine Wirbelschichttrocknung gemäß Stand der Technik.

### Allgemeine Herstellung eines Emulsionskonzentrates (EC)

Das organische Lösemittel wird vorgelegt. Unter Rühren werden anschließend alle Komponenten hinzugegeben (ohne besondere Reihenfolge). Es wird solange gerührt, bis eine klare Lösung entsteht.

### Allgemeine Herstellung eines organischen Disnerisionskonzentrats (OD):

Es wird zunächst das organische Lösemittel bei Raumtemperatur vorgelegt. Unter Rühren werden die Wirkstoffe sowie die weiteren Komponenten hinzugegeben (ohne besondere Reihenfolge). Es erfolgt eine Vorzerkleinerung mit einer Kolloidmühle, gefolgt von einer Nassvermahlung beispielsweise mittels einer Perlmühle.

### Bestimmung von 2-(1-Chlorocyclopropyl)-1-(2-chlorophenyl)-3-(1H-1,2,4-triazol-1-yl)propan-2-ol (Verbindung III) in Formulierungen

2-(1-Chlorocyclopropyl)-1-(2-chlorophenyl)-3-(1H-1,2,4-triazol-1-yl)propan-2-ol wird von den Formulierbestandteilen auf einer Umkehrphasensäule unter Verwendung eines isokratischen Laufmittels getrennt. Nach der MS/MS Detektion, wird die quantitative Auswertung durch Vergleich der Peakflächen mit denen des Referenzgegenstandes, unter Verwendung eines externen Standards durchgeführt.

| | |
|---|---|
| Hochdruckflüssigkeitschromatograph : | HP 1090 |
| Probeninjektion: | HP 1090 Autoinjector |
| Massenspektrometer : | Quattro I, Fisons |
| Integration und Auswertung : | MassLynx von Micromass |

Die Proben werden jeweils, soweit nicht anders angegeben, Licht ausgesetzt und 4 Wochen gelagert.

### Beispiel 1: Rezeptur einer Prothioconazole -Emulsionskonzentrat-Formulierung (Lagerung im Dunkeln)

| | **1a** | **1b** |
|---|---|---|
| **Komponente** | **% wt/wt** | |
| Prothioconazole | 12,75 | 13,00 |
| Bixafen | 6,37 | 6,5 |
| Fluopyram | 6,37 | 6,5 |
| Lucramul HOT 5902 | 7,50 | 7,50 |
| Lucramul CO 30 | 7,50 | 7,50 |
| Soprophor 796/P | 5,00 | 5,00 |
| SAG1572 | 0,10 | 0,10 |
| Genagen^{®} 4296 (Vergleich) | 54,41 | 43,90 |
| 9-Decensäure (Erfindungsgemäß) | | 10,00 |

| | **Verbindung (III)-Gehalt %wt/wt** |
|---|---|
| -Beispiel 1a (Vergleich) | 0,0037 |
| Beispiel 1b | 0,0023 |

### Beispiel 2: Rezeptur eines Prothioconazole -Supsenstionskonzentrats

| | **2a** | **2b** |
|---|---|---|
| **Komponente** | **% wt/wt** | |
| Prothioconazole | 45 | 45 |
| Pluronic PE 10500 | 6,0 | 6,0 |
| Harnstoff | 10 | 10 |
| Entschäumer (Momentive^{®} SAG1572) | 0,1 | 0,1 |
| Proxel GXL | 0,2 | 0,2 |
| Rhodopol G | 0,2 | 0,2 |
| Wasser | 28,5 | 28,5 |
| Wasser (Vergleich) | 10 | |
| 9-Decensäure | | 10 |

| | **Verbindung (III)-Gehalt %wt/wt** |
|---|---|
| -Beispiel 2a (Vergleich) | 0.0026 |
| Beispiel 2b | 0.0024 |

### Beispiel 3: Rezeptur eines Prothioconazole - WG (Wasserdispergierbares Granulat, Wirbelschichtgranulat)

| | **3a** | **3b** | **3c** |
|---|---|---|---|
| **Komponente** | **% wt/wt** | | |
| Prothioconazole | 50 | 50 | 50 |
| Baykanol SL | 15 | 15 | 15 |
| Oparyl MT 804 | 1 | 1 | 1 |
| Kaolin W | 29 | 29 | 29 |
| Kaolin W (Vergleich) | 5 | 5 | 5 |
| 9-Decensäure | | 5 | |
| 9-Decenoatester with 10 EO groups | | | 5 |

| | **Verbindung (III)-Gehalt %wt/wt** |
|---|---|
| -Beispiel 3a (Vergleich) | 0.0094 |
| Beispiel 3b | 0.0071 |
| Beispiel 3c | 0.0077 |

### Beispiel 4: Rezeptur eines Prothioconazole - WG (Wasserdispergierbares Granulat, hergestellt als Extrusionsgranulat)

| | **3a** | **3b** | **3c** |
|---|---|---|---|
| **Komponente** | **% wt/wt** | | |
| Prothioconazole | 50 | 50 | 50 |
| Baykanol SL | 20 | 20 | 20 |
| Pergopak M | 5 | 5 | 5 |
| NaHCO3 | 20 | 20 | 20 |
| NaHCO3 (Vergleich) | 5 | 5 | 5 |
| 9-Decensäure | | 5 | |
| 9-Decenoatester with 10 EO groups | | | 5 |

## Patentansprüche

1. Formulierungen enthaltend
a) Prothioconazole sowie
b) mindestens eine Verbindung der Formel (IV) 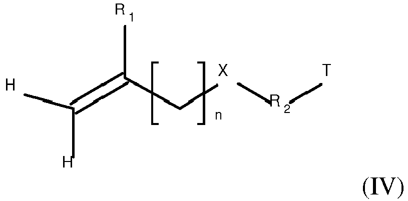 wobei
n = 0 - 24; bevorzugt 2-16; weiter bevorzugt 4-14; und besonders bevorzugt 8-12:
R₁ = H, C₁-C₆ Alkyl, -CN, Cl, Br, Fl, ; bevorzugt H, C₁-C₄ Alkyl, weiter bevorzugt H, Methyl; und besonders bevorzugt H;
X= -COO-, -CONR₃R₄, -S-, -SO₂-, -O- und -COS-; bevorzugt -COO-, CONR₃R₄, -S-, -O- und-COS-; weiter bevorzugt -COO-, CONR₃R₄; und besonders bevorzugt -COO-,;
R₂ = (-CH₂-)ₘ, (-CH₂O-)ₘ, (-C₂H₄O-)ₘ, (-C₃H₆O-)ₘ, (-C₄H₈O-)ₘ; bevorzugt (-CH₂-)ₘ, (-CH₂O-)ₘ, (-C₂H₄O-)ₘ, (-C₃H₆O-)ₘ; weiter bevorzugt (-CH₂O-)ₘ, (-C₂H₄O-)ₘ, (-C₃H₆O-)ₘ; und besonders bevorzugt (-CH₂O-)ₘ,
T= H, Sulfat, Phosphat, C₁-C₃ Alkyl, -OH, -SH, Sulfon; bevorzugt H, Sulfat, Sulfon, -OH, Phosphat; weiter bevorzugt H, Sulfat, -OH; und besonders bevorzugt H und OH;
R₃ = H, C₁-C₆ Alkyl, C₃-C₆ Cycloalkyl, C₆-C₁₀ Aryl, bevorzugt H, C₁-C₄ Alkyl, C5-C6 Cycloalkyl, Phenyl, weiter bevorzugt H, Methyl und Phenyl; und besonders bevorzugt H;
R₄ = H, methyl, ethyl propyl, (-CH₂-)ₘ, (-CH₂O-)ₘ, (-C₂H₄O-)ₘ, (-C₃H₆O-)ₘ, (-C₄H₈O-)ₘ; bevorzugt H, methyl, ethyl propyl, (-CH₂-)ₘ, (-CH₂O-)ₘ, (-C₂H₄O-)ₘ, (-C₃H₆O-)ₘ; weiter bevorzugt H, methyl, ethyl propyl, (-CH₂O-)ₘ, (-C₂H₄O-)ₘ, (-C₃H₆O-)ₘ; und besonders bevorzugt H, methyl, ethyl propyl, (-CH₂O-)ₘ,
und R₃ verschieden von R₄ ist, und
m = 0 - 100, bevorzugt 2 - 50, weiter bevorzugt 5 - 25, und besonders bevorzugt 8 - 12;
wobei die Formulierung frei von Verbindungen gemäß Formel (I) ist 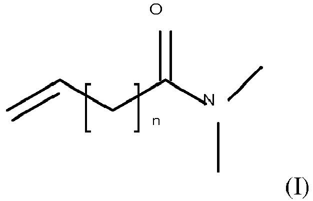 in welcher
n für 3, 4, 5, 6, 7, 8, 9, 10, 11, 12, 15 oder 18 steht,
und darüber hinaus frei von Glycosid-Tensiden mit 9-Decenoylrest,
wobei
bei X= -CNR₃R₄ und m=0 T entfällt.

2. Formulierungen gemäß Anspruch 1, **dadurch gekennzeichnet, dass** in der Formel (IV)
n = 2-16;
R₁ = H, C₁-C₄ Alkyl,
X= -COO-, CONR₃R₄, -S-, -O- und -COS-; R₂ = (-CH₂-)ₘ, (-CH₂O-)ₘ, (-C₂H₄O-)ₘ, (-C₃H₆O-)ₘ; T= H, Sulfat, Sulfon, -OH, Phosphat;
R₃ = H, C₁-C₄ Alkyl, C5-C6 Cycloalkyl, Phenyl;
R₄ = H, methyl, ethyl propyl, (-CH₂-)ₘ, (-CH₂O-)ₘ, (-C₂H₄O-)ₘ, (-C₃H₆O-)ₘ;
und R₃ verschieden von R₄ ist, und
m = 0 *-* 50; ist.

3. Formulierungen gemäß Anspruch 1, **dadurch gekennzeichnet, dass** in der Formel (IV)
n = 8-12:
R₁ = H;
X= -COO-,
R₂ = (-CH₂O-)ₘ,
T= H und OH;
m = 8 - 12; ist.

4. Formulierungen gemäß Anspruch 1, **dadurch gekennzeichnet, dass** in der Formel (IV)
n =7:
R₁ = H;
X= -COO-,;
T=H;
m = 0; ist.

5. Formulierung nach Anspruch 1, **dadurch gekennzeichnet, dass** Verbindung IV ausgewählt ist aus der Gruppe, die
9-Decensäure, 9-Decenoatester mit n EO Gruppen (EO= C₂H₄O), wobei n= 8,9,10,11, und bevorzugt n=10 ist;
C10-17 DMAPA-Amide 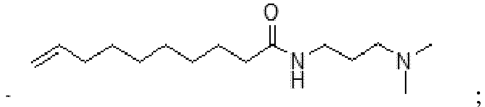 C10-20 DMAPA Amine-Oxide 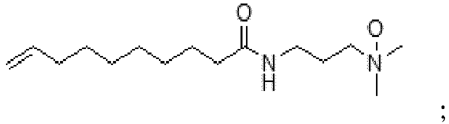 und
C10-24: Sulfobetaine 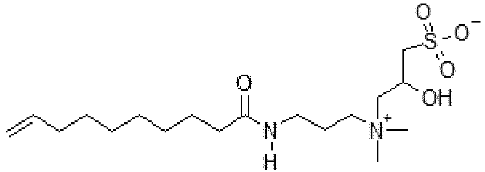 umfaßt.

6. Formulierungen nach einem der vorhergehenden Ansprüche, **dadurch gekennzeichnet, dass** der Anteil der Komponente b) 1 Gew.-% bis 50 Gew.-% beträgt.

7. Formulierungen nach einem der vorhergehenden Ansprüche, **dadurch gekennzeichnet, dass** der Anteil der Komponente a) 1 Gew.-% bis 50 Gew.-% beträgt.

8. Formulierungen nach einem der vorhergehenden Ansprüche, **dadurch gekennzeichnet, dass** diese weiterhin mindestens eine der folgenden Komponenten enthalten:
c)
c1: nicht-ionischer Dispergator/Emulgator,
c2: ionischer Dispergator/Emulgator
d) andere von a) verschiedene agrochemische Wirkstoffe
e) Lösemittel einsschlieplich OD Carrier
f) Träger (WGs, sowie Aerosile für SC/TK/WG)
g)
g1: organischer Verdicker
g2: anorganischer Verdicker
h) weitere Zusatz- und Hilfsstoffe

9. Formulierungen gemäß Anspruch 8, **dadurch gekennzeichnet, dass** diese einen nichtionischen (c1) und/oder anionischen (c2) Emulgator enthalten.

10. Formulierungen gemäß Anspruch 8, **dadurch gekennzeichnet, dass** agrochemische Wirkstoffe d) ein oder mehrere insektizide oder fungizide Wirkstoffe sind.

11. Formulierungen gemäß Anspruch 8, **dadurch gekennzeichnet, dass** der weitere agrochemische Wirkstoff d) Bixafen ist.

12. Formulierungen gemäß Anspruch 10, **dadurch gekennzeichnet, dass** diese höchstens 5 Gew.-% Wasser enthalten.

13. Verfahren zur Herstellung der Formulierungen gemäß einem der vorhergehenden Ansprüche durch Mischen der Komponenten a) und b) sowie den gegebenenfalls weiteren Additiven.

14. Verfahren zur Bekämpfung von schädlichen Organismen umfassend das in Kontaktbringen der schädlichen Organismen, ihres Habitats, ihrer Wirte, wie Pflanzen und Saatgut, sowie des Erdreichs, des Gebiets und der Umgebung in denen sie wachsen oder wachsen könnten, aber auch von Materialien, Pflanzen, Saatgut, Erdreich, Oberflächen oder Räumen, die vor dem Angriff oder dem Befall durch pflanzenschädigende Organismen geschützt werden sollen, mit einer wirksamen Menge der Formulierungen gemäß Anspruch 1.

15. Verwendung der Formulierungen gemäß einem der vorhergehenden Ansprüche zum Schutz von Pflanzen einschließlich Saatgut, insbesondere um Nutzpflanzen vor dem Befall durch schädliche Organismen zu schützen.

16. Verwendung der Formulierungen gemäß einem der vorhergehenden Ansprüche zur Bekämpfung pflanzenschädlicher Organismen, wie beispielsweise phytopathogene Schadpilzen, Insekten, Arachniden, Nematoden und Schadpflanzen.

17. Verwendung gemäß Ansprüche 15 oder 16, **dadurch gekennzeichnet, dass** die schädlichen Organismen phytopathogene Schadpilze sind.

18. Emulsion, erhältlich durch Mischen von Wasser mit Formulierungen gemäß Anspruch 1, wobei das Mischungsverhältnis von Wasser zu Emulsionskonzentrat im Bereich von 1000 zu 1 bis 1 zu 1 betragen kann.
